# EUROPÄISCHE PATENTSCHRIFT

(11) **EP 4 355 390 B1**
(45) Veröffentlichungstag und Bekanntmachung des Hinweises auf die Patenterteilung: **15.04.2026**
(21) Anmeldenummer: 22734251.6
(22) Anmeldetag: 13.06.2022
(51) Int. Cl.: A61M 5/158, A61M 5/32, A61M 25/00, A61M 25/06

(54) **SICHERHEITSKANÜLENANORDNUNG**
SAFETY NEEDLE ASSEMBLY
ASSEMBLAGE D'AIGUILLE DE SÉCURITÉ

(30) Priorität: 16.06.2021 DE 102021115556
(43) Veröffentlichungstag der Anmeldung: 24.04.2024
(73) Patentinhaber: Sarstedt AG & Co. KG, 51588 Nümbrecht (DE)
(72) Erfinder: HAUPT, Viktor, 51588 Nümbrecht (DE)
(74) Vertreter: Bauer PSU PartG mbB
(86) Internationale Anmeldenummer: PCT/EP2022/065995
(87) Internationale Veröffentlichungsnummer: WO 2022/263360

(56) Entgegenhaltungen:
- EP-A1- 2 343 095
- EP-A1- 2 985 049
- US-A- 5 746 215
- US-A1- 2019 167 896

## Beschreibung

Die Erfindung betrifft eine Sicherheitskanülenanordnung umfassend:
a) eine Kanüle zur Punktion menschlichen oder tierischen Gewebes, wobei die Kanüle in einem distalen Endabschnitt eine Spitze aufweist,
b) einen Gleitkörper, der in einem distalen Endabschnitt mit der Kanüle und in einem proximalen Endabschnitt mit einem flexiblen Schlauch versehen ist, wobei eine sich durch den Gleitkörper erstreckende Strömungsverbindung zwischen der Spitze der Kanüle und einem proximalen Ende des Schlauchs besteht,
c) einen Grundkörper, in dem der Gleitkörper von einer Gebrauchsstellung, in der sich die Spitze der Kanüle außerhalb des Grundkörpers befindet, in eine Sicherungsstellung verlagerbar ist, in der sich die Spitze der Kanüle innerhalb des Grundkörpers befindet, wobei der Grundkörper aus mindestens drei Grundkörperteilen zusammengesetzt ist, von denen
   - ein erstes Grundkörperteil hülsenförmig ausgebildet ist und einen distalen Endabschnitt des Gleitkörpers in dessen Gebrauchsstellung umschließt,
   - ein zweites Grundkörperteil sich in proximale Richtung an das erste Grundkörperteil anschließt und mit diesem verbunden ist, wobei das zweite Grundkörperteil in einem Querschnitt U-förmig, L-förmig, C-förmig oder deckelförmig ausgebildet ist und
   - ein drittes Grundkörperteil und das zweite Grundkörperteil zu einem an beiden gegenüberliegenden Stirnseiten offenen Hohlkörper zusammenfügbar sind, wobei das dritte Grundkörperteil deckelförmig, C-förmig, L-förmig oder U-förmig ausgebildet und über ein Filmscharnier mit dem zweiten Grundkörperteil oder dem ersten Grundkörperteil verbunden ist.
d) ein zwischen dem Grundkörper und dem Gleitkörper angeordnetes Stellelement, mittels dessen der Gleitkörper von der Gebrauchsstellung in die Sicherungsstellung verlagerbar ist und
e) ein an dem Grundkörper angeordneten Auslösemechanismus, mittels dessen die Verlagerung des Gleitkörpers von der Gebrauchsstellung in die Sicherungsstellung auslösbar ist, wobei der Auslösemechanismus mindestens ein Auslöseglied aufweist, das mittels eines Fingers einer die Sicherheitskanülenanordnung gebrauchenden Person mit einer radial gerichteten Druckkraft oder mittels zweier Finger mit einem Drehmoment beaufschlagbar ist und das mit einem Verriegelungselement versehen ist, das infolge einer Beaufschlagung mit der Druckkraft oder dem Drehmoment von einer Verriegelungsstellung, in der es mit dem Gleitkörper in Eingriff ist und diesen in der Gebrauchsstellung verriegelt, in eine Auslösestellung verlagerbar ist, in dem es außer Eingriff mit dem Gleitkörper ist, sodass sich dieser in die Sicherungsstellung begibt,

Im Sinne der vorliegenden Erfindung ist unter der Lage- bzw. Richtungsbezeichnung "proximal" eine zum Körper der die Sicherheitskanülenanordnung gebrauchenden Person, insbesondere zu deren die Sicherheitskanülenanordnung haltenden Hand, hin gerichtete Anordnung oder Lage zu verstehen, wohingegen mit "distal" eine entsprechend vom Körper bzw. der Hand weggerichtete Anordnung oder Lage gemeint ist. Folglich ist ein proximales Ende eines Gegenstandes, also auch der hier in Rede stehenden Sicherheitskanülenanordnung, näher an dem Körper der Person angeordnet als ein distales.

Kanülenanordnungen werden in der Medizin z.B. bei der Entnahme von Körperflüssigkeiten, insbesondere für die venöse Blutentnahme oder für die Infusion von Flüssigkeiten in ein Gefäß des menschlichen Körpers verwendet. In diesem Zusammenhang dienen Sicherheitskanülenanordnungen, die eine Schutzanordnung für die Kanüle aufweisen, dazu, Nadelstichverletzungen auf bestmögliche Weise zu verhindern und dabei gleichwohl für die die Kanülenanordnung gebrauchende Person einen größtmöglichen Komfort zu bieten, sowie für den Patienten beim Gebrauch der Kanülenanordnung einschließlich der Aktivierung des Nadelschutzes Schmerzen und Traumata zu verhindern. Während bei einem passiven Nadelschutz die Rückzugsbewegung der Nadel nach Abschluss der Blutentnahme oder Infusion automatisch und ohne weitere Einflussnahme durch die gebrauchende Person erfolgt, ist bei einem aktiven Nadelschutz eine bewusste Interaktion durch die die Kanülenanordnung gebrauchende Person erforderlich, um die Kanüle in ihre Sicherungsstellung zu überführen. Die vorliegende Erfindung bezieht sich in erster Linie auf Sicherheitskanülenanordnungen mit aktivem Nadelschutz.

Der "Grundkörper" im Sinne der vorliegenden Anmeldung ist so definiert, dass er eine Gehäusefunktion erfüllt und dazu einen von ihm umschlossenen Innenraum besitzt, in dem der Gleitkörper in der Gebrauchsstellung zumindest teilweise untergebracht ist. In der Sicherungsstellung, d.h. nach Verlagerung des Gleitkörpers, ist zumindest die Kanüle selbst einschließlich ihrer Spitze vollständig in dem Innenraum des Grundkörpers untergebracht, um einerseits Stichverletzungen durch die Spitze und andererseits Berührungen auch mit der übrigen Nadel, an der sich kontaminierte oder infektiöse Körpersubstanz befinden könnte, sicher zu verhindern. Unter "Grundkörper" sind im vorliegenden Fall sowohl einteilige als auch mehrteilige Gestaltungen denkbar, wobei im Falle einer Mehrteiligkeit die einzelnen Grundkörperteile ineinander gesteckt oder geclipst oder sonstwie miteinander verbunden werden können. Auch die Anbindung mehrerer Grundkörperteile aneinander mit Hilfe so genannter Filmscharniere ist im Rahmen der vorliegenden Anmeldung denkbar. Wandungen des Grundkörpers müssen in diesem Zusammenhang nicht vollständig geschlossen sein, sondern können auch Durchbrüche, Unterbrechungen, Schlitze oder sonstige Perforationen besitzen, so dass nicht zwingend eine hermetische Abschirmung des Innenraums zur Umgebung hin vorliegen muss.

Unter einem "Stellelement" im Sinne der vorliegenden Erfindung ist ein Element zu verstehen, das eine Art "Antrieb" eine erforderliche Kraft auf den Gleitkörper auszuüben vermag, um diesen von der Gebrauchsstellung in die Sicherungsstellung zu überführen. Entweder können "Antriebselement" und zugehöriger "Energiespeicher" getrennt voneinander sein. Besonders zweckmäßig ist allerdings die Kombination dieser beiden Funktionen in einem Bauelement, insbesondere in Form eines "Federelements", bei dem entweder mechanische Energie gespeichert ist (z.B. Schraubenfeder o.ä.) oder Druckenergie, wie beispielsweise in einem vorgespannten Gasspeicher.

Unter einem "Gleitkörper" soll in dieser Anmeldung ein Bauelement verstanden werden, das an einem distalen Ende die Kanüle aufnimmt und an einem proximalen Ende mit dem Schlauch verbunden ist. Eine Bezeichnung als "Kanülenhalter" oder "Nadelträger" ist ebenfalls gebräuchlich.

### Stand der Technik

Aus der EP 2 108 394 B1 ist eine Schutzabdeckungsanordnung für eine Kanüle bekannt, bei der der Grundkörper aus drei Grundkörperteilen zusammengesetzt ist. Ein erstes Grundkörperteil wird von einem distalen, hülsenförmigen Endabschnitt gebildet, in dem die Kanüle in der zurückgezogenen, d.h. Sicherungsstellung des Gleitkörpers, untergebracht ist, wodurch Nadelstichverletzungen vermieden werden sollen. Das zweite Grundkörperteil ist einstückig und starr (d.h. ohne ein Gelenk oder Scharnier) mit dem hülsenförmigen ersten Grundkörperteil verbunden und besitzt zunächst die Form einer halbzylindrischen Rinne, um sich dann am proximalen Ende trapezförmig zu erweitern. Auch in dem Erweiterungsabschnitt ist das zweite Grundkörperteil - wenn auch mit vergrößerter Breite - rillen- oder wannenförmig. Eine proximale Stirnwand weist einen halbkreisförmigen Ausschnitt auf. Die bekannte Anordnung weist darüber hinaus ein drittes Grundkörperteil auf, das über ein parallel zu der Längsachse des Gleitkörpers verlaufendes Filmscharnier mit dem zweiten Grundkörperteil verbunden ist. Das Filmscharnier befindet sich in dem verbreiterten proximalen Endabschnitt des zweiten Grundkörperteils. Der dritte Grundkörperteil weist eine zu dem Endabschnitt des zweiten Grundkörperteils komplementäre rillen bzw.- wannenförmige Gestalt auf. Im zusammengefügten Zustand des zweiten und dritten Grundkörperteils liegen diese im breitenmäßig erweiterten Endabschnitt des zweiten Grundkörperteils komplementär aufeinander auf und sind über eine Schnapphakenverbindung gegeneinander fixiert. Das dritte Grundkörperteil weist ebenfalls in seiner proximalen Stirnwand eine halbkreisförmige Ausnehmung auf, die sich mit der halbkreisförmigen Ausnehmung in der proximalen Stirnwand des zweiten Grundkörperteils zu einem kreisförmigen Durchlass für den flexiblen Schlauch bzw. den Gleitkörper selbst ergänzt. Nachteilig an dieser bekannten Sicherheitsnadelanordnung ist der Umstand, dass der Grundkörper in einem Mittelbereich zwischen dem hülsenförmigen ersten Grundkörperteil und der distalen Stirnfläche des dritten Grundkörperteils offen ist, da er dort lediglich aus der Halbschale des zweiten Grundkörperteils besteht. Daher besteht die Gefahr, dass nach Benutzung der Vorrichtung und Überführung des Nadelträgers in die Sicherungsstellung eine Kontamination von Personen durch Berühren der mit Körperflüssigkeit behafteten, frei zugänglichen Kanüle (außerhalb des Spitzenbereichs derselben) stattfindet. Darüber hinaus weist die bekannte Sicherheitskanülenanordnung kein Stellelement auf, mit dem der Gleitkörper selbsttätig durch bloßes manuelles Auslösen von der Gebrauchsstellung in die Sicherungsstellung überführt werden könnte. Eine sehr ähnliche Sicherheitskanülenanordnung ist darüber hinaus aus der auf dieselbe Anmelderin zurückgehenden WO 2013/068855 A1 bekannt.

Außerdem beschreibt die EP 2 509 674 B1 einen Grundkörper einer Sicherheitskanülenanordnung, bei der zwei Grundkörperhälften mittels eines quer zu einer Längsachse des Grundkörpers verlaufenden und zwischen zwei Scharnierabschnitten eine Durchtrittsöffnung für die Kanüle freilassenden Filmscharnier verbunden sind. Im zusammengefügten Zustand der beiden Grundkörperteile befindet sich in den gegenüber liegenden Schmalseiten des Grundkörpers jeweils ein in Längsrichtung verlaufender Schlitz, durch den jeweils ein mit dem Nadelträger verbundener Griffflügel hindurchtritt und in axiale Richtung gemeinsam mit dem Nadelträger verschiebbar ist. Ein Stellelement für eine selbsttätige Überführung des Gleitkörpers von der Gebrauchs- in die Sicherungsstellung ist bei der bekannten Sicherheitsnadelanordnung nicht vorgesehen.

Darüber hinaus offenbart die WO 2011/100039 A1 eine Sicherheitskanülenanordnung, bei der an das hintere Ende des Gleitkörpers bzw. Kanülenträgers kein Schlauch angeschlossen ist, sondern bei der die Nadel beidseitig des Gleitkörpers vorsteht und das hintere, freie Ende der Nadel dazu dient, dass darauf unmittelbar ein Blutentnahmeröhrchen aufgesteckt wird. Deshalb ist der Gleitkörper gemäß der WO 2011/100039 A1 so ausgebildet, dass er einen hinreichend großen Durchmesser für das Einführen des Blutentnahmeröhrchens besitzt, und am proximalen Ende offen ist, um überhaupt ein Einführen zu ermöglichen.

Die Sicherheitsfunktion der vorbekannten Kanülenanordnung ergibt sich dadurch, dass nach Beenden des Blutentnahmevorgangs und nach dem Abkoppeln und Herausziehen des Blutentnahmeröhrchens aus dem Grundkörper ein zuvor in einer "Wartestellung" außerhalb des Grundkörpers befindlicher Aufnahmekörper für den Gleitkörper und die darin gelagerte Doppelkanüle in eine Richtung quer zu der Längsachse der Kanüle so in den Raum für das nicht mehr dort befindliche Blutentnahmeröhrchen bewegt wird, dass ein Auslöseglied eine Rückzugsbewegung der Kanüle samt Gleitkörper dann freigibt, wenn ein Aufnahmequerschnitt des Aufnahmekörpers über das proximale freie Kanülenende geschoben wurde und somit eine Rückzugsbewegung des Gleitkörpers samt der Kanüle im Inneren des Aufnahmekörpers ermöglicht. Aufnahmekörper und Grundkörper befinden sich dabei in einer zueinander geneigten (geknickten) Anordnung, was allerdings aufgrund des hinreichend kleinen Winkels zwischen den beiden Längsachsen für die erforderliche Bewegung des Gleitkörpers auf einer Kurvenbahn unschädlich ist.

Das Auslöseglied und das damit verwirklichte Verriegelungsprinzip umfasst ein Schlüsselloch, das mit seinem schmalen Querschnitt eine Verriegelung des Gleitkörpers in der Gebrauchsstellung bewirkt, hingegen mit seinem vergrößerten Querschnitt in der Auslösestellung einen Hindurchtritt des Gleitkörpers gestattet. Auch wenn in der WO 2011/100039 A1 ein Filmscharnier zur Verbindung zweier Grundkörperteile offenbart ist, eignet sich die vorbekannte Konstruktion nicht zur Verwirklichung einer Mehrzahl von Grundkörperteilen und deren Verbindung jeweils mittels eines Filmscharniers.

In der US 5,746,215 A ist eine Sicherheitskanülenanordnung beschrieben, bei der die Kanüle erst aus einer initial eingenommenen Sicherungsstellung mittels eines von außen zugänglichen Schiebers in eine Gebrauchsstellung herausgezogen werden muss. Bei dieser Überführung aus dem Grundkörper heraus wird ein Federelement gelängt und damit vorgespannt, das sich nach Beendigung der Blutentnahme bzw. Punktion durch Druck auf ein Auslöseglied wieder zusammenzieht und dabei den Gleitkörper samt Kanüle in die Sicherungsstellung rücküberführt.

Der EP 2 985 049 A1 ist eine Schmetterlingsnadel-Schutzhülle entnehmbar, die einen Grundkörper zur Aufnahme einer Schmetterlingsnadel umfasst, der als einstückiges Spritzgussteil ausgebildet ist. Die Schmetterlingsnadel weist einen Nadelabschnitt, einen Flügelabschnitt, der aus dem Grundkörper hervorsteht, und einen an dem Flügelabschnitt befestigten Griffabschnitt auf. Dabei ist ein Ende des Griffabschnitts mit dem Nadelabschnitt verbunden und das andere Ende zum Verbinden mit einem Schlauch vorgesehen. Die Schmetterlingsnadel ist zwischen einem Gebrauchszustand mit vorstehender Nadelspitze und einem Sicherungszustand ohne vorstehende Nadelspitze verschiebbar. Gemäß der EP 2 985 049 A1 umfasst der Grundkörper einen Nadelspitzen-Schutzkopf, eine obere Abdeckung und eine untere Abdeckung umfasst. Der Nadelspitzen-Schutzkopf ist als ein Zylinder mit einem axial durchgängigen Nadelloch ausgeführt. Die obere Abdeckung und die untere Abdeckung werden von länglichen Bauteilen gebildet, die sich in einer axialen Richtung des Grundkörpers erstrecken und jeweils ein distales und ein proximales Ende aufweisen. Dabei sind die distalen Enden der oberen Abdeckung und der unteren Abdeckung durch Filmscharniere mit dem zylinderförmigen Schutzkopf des Grundkörpers verbunden.

Schließlich offenbart die EP 1 448 260 A1 noch ein Abdeckgehäuse einer Flügelkanüle, bei der das Gehäuse (Grundkörper) aus zwei komplementären und über ein Filmscharnier verbundenen Halbschalen zusammengesetzt ist. Die Verbindung der beiden Halbschalen erfolgt über ein parallel zu der Längsachse des Abdeckgehäuses bzw. des Nadelträgers verlaufenden Filmscharnier. Auf einer dem Filmscharnier gegenüber liegenden Längsseite des Gehäuses sind Schnapphaken zur Festlegung beider Gehäusehälften in dem geschlossenen Zustand angeordnet. Auch diese vorbekannte Gehäusekonstruktion weist an zwei gegenüber liegenden Längsseiten in Richtung der Längsachse verlaufende Schlitze auf, die eine Verschiebung des Gleitkörpers samt der daran angeordneten und in entgegengesetzte Richtungen abstehenden Halteflügel erlaubt.

### Aufgabe

Der Erfindung liegt die Aufgabe zugrunde, eine Sicherheitskanülenanordnung vorzuschlagen, bei der der Vorgang des Zusammenbauens erleichtert und insbesondere die Positionierung des Stellelements und die Erzeugung von dessen Vorspannung erleichtert wird. Gleichwohl soll der Herstellungsaufwand reduziert werden.

### Lösung

Ausgehend von einer Sicherheitskanülenanordnung der eingangs beschriebenen Art wird die zugrunde liegende Aufgabe dadurch gelöst, dass sich das Stellelement mit seinem distalen Ende an einer Stützfläche des ersten Grundkörperteils abstützt.

Die erfindungsgemäße Kanülenanordnung, bei der es sich vorzugsweise um eine Blutentnahmevorrichtung handelt, bietet große Vorteile bei der Montage, insbesondere beim Einsetzen des Gleitkörpers in den Grundkörper. Hierzu werden zunächst das erste Grundkörperteil und das sich daran anschließende zweite Grundkörperteil, die beide in Richtung der Längsachse des Grundkörpers bzw. Gleitkörpers betrachtet axial hintereinander angeordnet sind, bereitgestellt oder - im Falle einer Filmscharnierverbindung zwischen dem ersten und dem zweiten Grundkörperteil - in eine lineare Ausrichtung zueinander gebracht. Sodann werden der Gleitkörper und das Stellelement zusammengefügt und als Einheit in das erste und zweite Grundkörperteil eingelegt oder aber es wird zunächst das (ungespannte) Stellelement in das erste und zweite Grundkörperteil eingesetzt und erst in einem nächsten Schritt wird der Gleitkörper hinzugefügt und mit diesem das Stellelement in den vorgespannten Zustand überführt.

Insbesondere im Hinblick auf eine sichere und passgenaue Positionierung des Stellelements in dem Grundkörper bietet das hülsenförmige erste Grundkörperteil einen großen Vorteil. Insbesondere im Falle einer Schraubenfeder als Stellelement lässt sich dieses koaxial zu dem hülsenförmigen ersten Grundkörperteil ausrichten und einsetzen und ist sodann während des Vorgangs der Überführung in den vorgespannten Zustand an seinem distalen Ende sicher gegen Verrutschen oder Ausweichen und Wegspringen fixiert.

Als letzter Schritt in der Montagefolge wird sodann das dritte Grundkörperteil mit dem zweiten Grundkörperteil verbunden, wodurch der Grundkörper komplettiert wird und in seiner endgültigen Form vorliegt.

Zur Vereinfachung der Handhabung bzw. um eine alternative Handhabung zusätzlich zu einem Ergreifen des gehäuseartigen Teils des Grundkörpers anzubieten, wird vorgeschlagen, dass der Grundkörper von zwei gegenüber liegenden Seiten mit jeweils einem Griffflügel versehen ist. Die Griffflügel können aus einem gummielastischen Material bestehen, um in bekannter Weise durch Ergreifen mit zwei Fingern in einem Bereich oberhalb des Grundkörpers gegeneinander gedrückt zu werden, bis sie miteinander in Kontakt stehen. Eine Anwendung mit zwei Griffflügeln führt zu einem erhöhten Komfort bei der Anwendung. Vorstellbar ist auch eine Anwendung mit nur einem Griffflügel oder auch gar keinem Griffflügel.

Eine Weiterbildung der Erfindung besteht darin, dass das Stellelement sich mit seinem distalen Ende an einer in einem Innenraum des ersten Grundkörperteils ausgebildeten Stufe oder an einer einen distalen Abschluss des ersten Grundkörperteils bildenden Stirnwand abstützt. Hierdurch wird eine besonders einfache und sichere Fixierung des Stellelements während des Überführens desselben in vorgespannten Zustand gewährleistet.

Die erfindungsgemäße Anordnung weiter ausgestaltend können die Griffflügel an ein hülsenförmiges Verbindungsteil angeschlossen sein, das auf den Grundkörper aufgeschoben ist. Dabei kann vorzugsweise ein Überlappungsbereich zwischen dem Verbindungsteil und dem Grundkörper sowohl zumindest einen axialen Abschnitt des ersten Grundkörperteils als auch jeweils einen axialen Abschnitt des zweiten Grundkörperteils und des dritten Grundkörperteils umschließen. Das hülsenförmige Verbindungsteil bewirkt auf diese Weise nicht nur einen sicheren und festen Anschluss der Griffflügel an den Grundkörper, sondern stabilisiert diesen nach Art einer Manschette bzw. Schelle, was aufgrund des Zusammensetzens des Grundkörpers aus mehreren Grundkörperteilen eine zusätzliche Sicherung des zusammengefügten Zustands bewirkt.

Eine erste Variante der Ausgestaltung des ersten und zweiten Grundkörperteils besteht darin, dass diese beiden Teile starr miteinander verbunden sind. Es liegt somit keine Gelenkigkeit zwischen diesen beiden Grundkörperteilen vor, insbesondere ist kein Filmscharnier zwischen diesen beiden Grundkörperteilen vorhanden. Bei dieser Variante ist die Ausrichtung des ersten Grundkörperteils zu dem zweiten stets automatisch gewährleistet und bei der Montage fällt ein Freiheitsgrad, der durch entsprechende Fixierung und Führung der Bauteile ausgeschaltet werden müsste, von vorneherein weg.

Alternativ zu der vorgenannten Variante ist es ebenfalls möglich, das erste Grundkörperteil und das zweite Grundkörperteil über ein Filmscharnier miteinander zu verbinden. In diesem Fall sollte eine Scharnierachse des Filmscharniers senkrecht zu und in einem Abstand zu der Längsachse des Gleitkörpers ausgerichtet sein.

In Bezug auf das dritte Grundkörperteil sind wiederum zwei grundsätzliche Fälle der Anbindung an die übrigen Grundkörperteile denkbar: Zum einen können das dritte Grundkörperteil und das zweite Grundkörperteil mittels eines Filmscharniers miteinander verbunden sein. Die Scharnierachse des Filmscharniers kann dabei parallel oder senkrecht zu der Längsachse des Gleitkörpers ausgerichtet sein. Bei dieser Variante bildet das zweite Grundkörperteil das mittlere Element einer aus den drei Grundkörperteilen gebildeten "Dreierkette", deren Verbindung über zwei Filmscharniere erfolgt, wobei das zweite Grundkörperteil mit beiden Filmscharnieren verbunden ist.

Alternativ zu der vorstehend genannten Ausführungsform kommt auch die Möglichkeit in Betracht, dass das dritte Grundkörperteil und das erste Grundkörperteil mittels eines Filmscharniers miteinander verbunden sind. In diesem Falle sollte - wie auch bereits im Fall der Verbindung zwischen dem ersten und dem zweiten Grundkörperteil der Fall - die Scharnierachse des Filmscharniers senkrecht zu und in einem Abstand zu der Längsachse des Gleitkörpers ausgerichtet sein. In letztgenanntem Fall lässt sich eine symmetrische Anordnung schaffen, bei der das erste Grundkörperteil das mittlere Glied der "Dreierkette" bildet und die beiden Endglieder der Kette von vorzugsweise identisch gestalteten Grundkörperteilen gebildet werden. Bei dieser Konstellation werden während des Fügevorgangs das zweite und das dritte Grundkörperteil vorzugsweise synchron um die jeweiligen Filmscharnierachsen auf die Längsachse des zuvor in das erste Grundkörperteil eingefügten Gleitkörpers zu geschwenkt und miteinander in Kontakt gebracht und gegeneinander fixiert.

Aus Symmetriegründen und zur Vereinfachung der Montage sollten in letztgenanntem Fall die beiden Filmscharniere, in axiale Richtung betrachtet, denselben Abstand von der Spitze der Kanüle aufweisen. Ebenso sollte - aus denselben Gründen - die beiden Filmscharniere jeweils denselben Abstand zu der Längsachse des Gleitkörpers besitzen.

Die Erfindung weiter ausgestaltend ist darüber hinaus vorgesehen, dass das erste Grundkörperteil
- in einem distalen Abschnitt rotationssymmetrisch und/oder
- in einem proximalen Abschnitt quaderförmig
ausgebildet ist, wobei vorzugsweise an gegenüber liegenden proximalen Kanten des quaderförmigen proximalen Abschnitts jeweils ein Filmscharnier angeordnet ist. Eine derartige Ausgestaltung ermöglicht die sichere Unterbringung des distalen Endes des Stellelements bzw. Gleitkörpers in dem rotationssymmetrischen distalen Abschnitt und eine Verdrehsicherung des Gleitkörpers in dem quaderförmigen proximalen Abschnitt, sofern der Gleitkörper eine Querschnittsform besitzt, die eine Verdrehung innerhalb des vorzugsweise einen rechteckförmigen, insbesondere quadratischen Querschnitt aufweisenden proximalen quaderförmigen Abschnitts unterbindet.

Eine bevorzugte Ausgestaltung der erfindungsgemäßen Sicherheitskanülenanordnung ist darin zu sehen, dass der rotationssymmetrische Abschnitt des ersten Grundkörperteils einen zylindrischen Spitzenabschnitt und einen ebenfalls zylindrischen Übergangsabschnitt zur Aufnahme des in der Gebrauchsstellung komprimierten Stellelements besitzt, wobei vorzugsweise auf den Spitzenabschnitt ein abziehbarer rohrförmiger Kanülenschutz aufgeschoben ist.

Schließlich wird noch vorgeschlagen, dass das zweite Grundkörperteil und das dritte Grundkörperteil in einem zusammengefügten Zustand gemeinsam
- einen sich in proximale Richtung an die Filmscharniere anschließenden Griffbereich ausbilden, der vorzugsweise auf seinem Außenmantel mit Rippen oder einer Vielzahl von Erhebungen zur Erhöhung der Griffigkeit ausgestattet ist und/oder
- einen sich in proximale Richtung an den Griffbereich anschließenden Auslösebereich ausbilden, der einen Auslösemechanismus aufweist und/oder
- einen an einem proximalen Ende des Grundkörpers angeordneten Verschlussbereich, in dem das zweite Grundkörperteil und das dritte Grundkörperteil mittels Schnapphaken und/oder Rastnasen formschlüssig miteinander verbunden sind.

Außerdem sieht eine Weiterbildung der Erfindung noch vor, dass das erste Grundkörperteil ein distales Ende des Grundkörpers bildet.

### Ausführungsbeispiel

Die Erfindung wird nachfolgend anhand eines Ausführungsbeispiels, das in der Zeichnung dargestellt ist, näher erläutert:

Es zeigt:
- Fig. 1 und 2:: Eine Sicherheitskanülenanordnung aus zwei verschiedenen Perspektiven mit einem distal aufgesteckten Kanülenschutz,
- Fig. 3:: eine Explosionszeichnung der Sicherheitskanülenanordnung,
- Fig. 4:: eine perspektivische Ansicht des Grundkörpers mit daraus hervortretender Kanüle,
- Fig. 5:: wie Figur 4, jedoch in einem Teilabschnitt des Grundkörpers,
- Fig. 6:: ein freigeschnittenes erstes Grundkörperteil mit darin eingeschobenem Gleitkörper,
- Fig. 7:: der Gleitkörper mit Kanüle, Stellelement und einem Abschnitt eines flexiblen Schlauchs,
- Fig. 8:: wie Figur 7, jedoch ohne das Stellelement,
- Fig. 9:: eine perspektivische Ansicht des Grundkörpers mit seitlich weggeklapptem zweiten und dritten Grundkörperteil,
- Fig. 10:: eine perspektivische Ansicht des ersten und des zweiten Grundkörperteils,
- Fig. 10a:: wie Fig. 10, jedoch aus einer anderen Perspektive
- Fig. 11 und 12:: einen Längsschnitt durch die Sicherheitskanülenanordnung gemäß den Figuren 1 und 2 mit dem Gleitkörper in Gebrauchsstellung,
- Fig. 12a:: eine Ausschnittsvergrößerung aus Fig. 12
- Fig. 13 bis 16:: jeweils einen Schnitt entlang der Linie I-I durch die Sicherheitskanülenanordnung gemäß den Figuren 1 und 2 in unterschiedlichen Stellungen der Auslöseglieder und Verriegelungselemente,
- Fig. 17:: eine perspektivische Ansicht der Sicherheitsnadel in einem Schnitt entlang der Linie II-II in den Figuren 1 und 2,
- Fig. 18:: einen Auslösemechanismus in einem Schnitt entlang der Linie III-III wie in Figur 1 in proximaler Blickrichtung,
- Fig. 19:: einen Längsschnitt durch die Sicherheitsnadelanordnung gemäß den Figuren 1 und 2 in der Sicherungsstellung der Gleitkörpers,
- Fig. 19a:: eine Ausschnittsvergrößerung aus Fig. 19
- Fig. 20:: einen Schnitt durch die Sicherheitskanülenanordnung gemäß den Figuren 1 und 2 entlang der Linie IV-IV mit dem Gleitkörper in der Sicherungsstellung in distaler Blickrichtung,
- Fig. 21:: wie Figur 18, jedoch in der Sicherungsstellung des Gleitkörpers, und
- Fig. 22:: ein Querschnitt durch die Sicherheitskanülenanordnung gemäß den Figuren 1 und 2 entlang der Line V-V in distaler Blickrichtung.

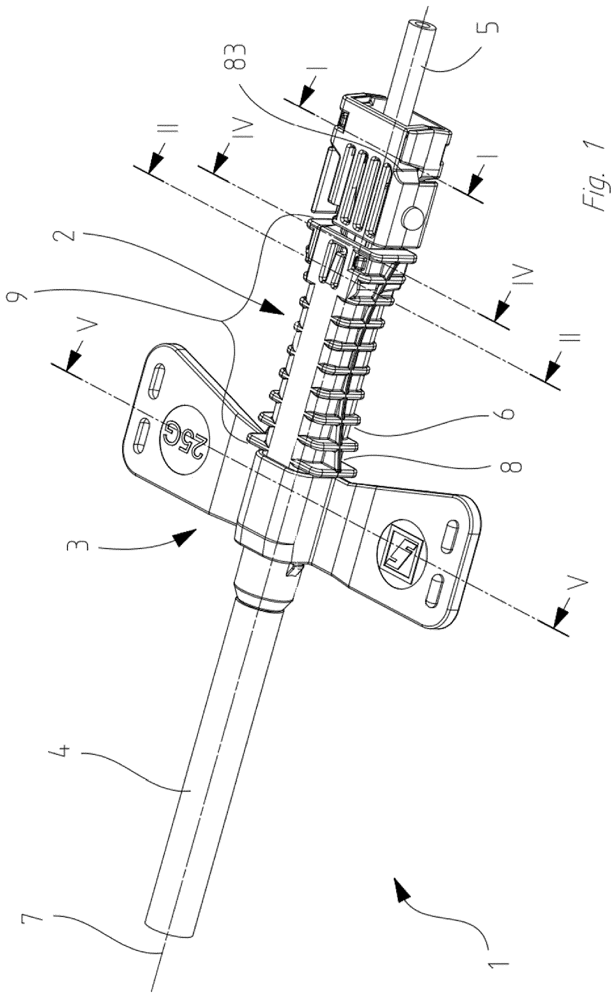

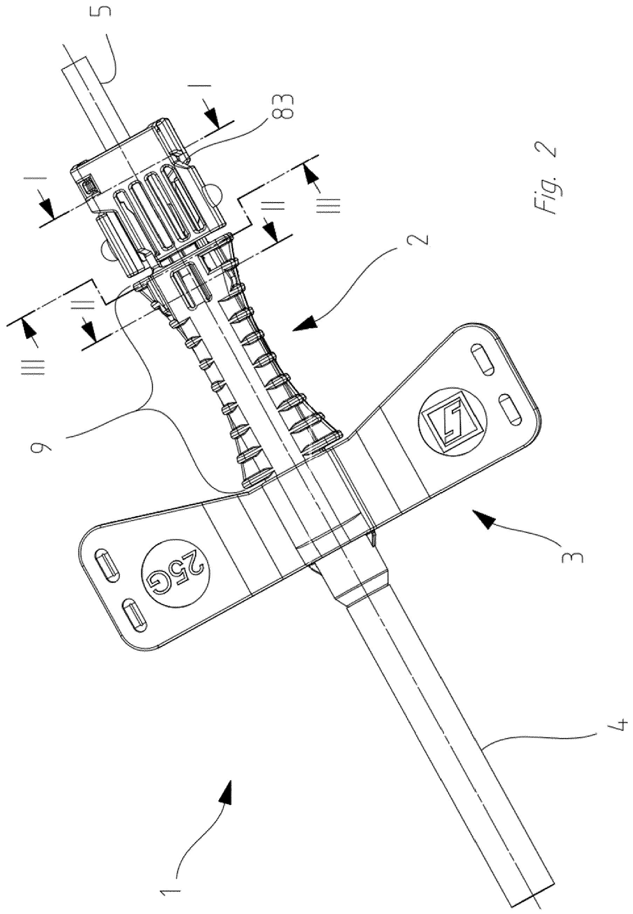

Von einer in den Figuren 1 und 2 in unterschiedlichen Perspektiven dargestellten Sicherheitskanülenanordnung 1 ist im dargestellten Auslieferungszustand ein ein langgestrecktes Gehäuse bildender Grundkörper 2, ein daran angeordnetes Flügelmodul 3, ein über eine distal vorstehende Kanüle geschobener rohrförmiger Kanülenschutz 4 sowie ein der Einfachheit halber abgeschnitten dargestellter flexibler Schlauch 5 zu erkennen. Der abgeschnitten dargestellte Schlauch 5, der an einen proximalen Endabschnitt eines in den Figuren 1 und 2 nicht sichtbaren Gleitkörpers angeschlossen ist, besitzt eine Länge von ca. 5 cm bis 30 cm und ist an seinem proximalen Ende mit einem Adapter ausgestattet ist, der die Konnektivität zu weiteren Handhabungsmitteln der Blutentnahme sicherstellt . Der Grundkörper 2 ist in einem Griffbereich 9 auf zwei gegenüber liegenden Seiten mit einer Mehrzahl von senkrecht zu einer Längsachse 7 des Grundkörpers (bzw. auch der Sicherheitskanülenanordnung insgesamt bzw. des Gleitkörpers) verlaufenden Querrippen 6 versehen, deren Überstand über die im Übrigen abgerundet quaderförmige Grundgeometrie des Griffbereichs 9 des Grundkörpers 2 ausgehend von einem maximalen Überstand bei den Querrippen 6 angrenzend an das Flügelmodul 3 zu dem proximalen Ende des Griffbereichs 9 des Grundkörpers 2 hin zunächst abnimmt, um sodann wieder anzuwachsen. Die äquidistant und parallel zueinander verlaufenden Querrippen 6 sind auf jeder Seite durch eine in einer Symmetrieebene des Grundkörpers 2 angeordnete Längsrippe 8 miteinander verbunden und stabilisiert. Auf diese Weise wird für zwei von gegenüber liegenden Seiten das Griffbereichs 9 des Grundkörpers 2 ergreifende Finger einer die Sicherheitskanülenanordnung 1 gebrauchenden Person ein besonders sicherer und ergonomischer Griff ermöglicht und somit die Handhabung vereinfacht.

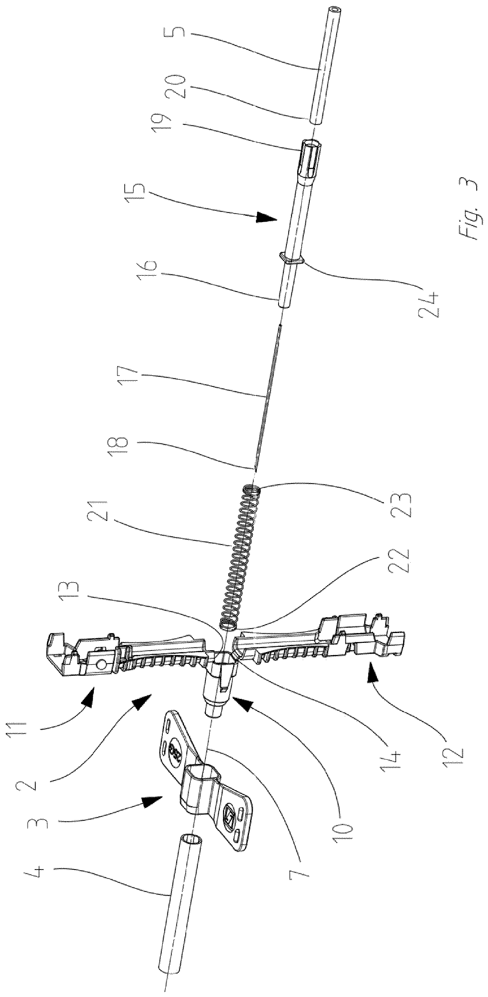

Aus der Explosionsdarstellung gemäß Figur 3 ist zu entnehmen, dass der Grundkörper 2 aus drei Grundkörperteilen 10, 11 und 12 zusammengesetzt ist, und zwar aus einem hülsenförmigen ersten Grundkörperteil 10, das sich am weitesten in distale Richtung erstreckt, und zwei halbschalenförmigen Grundkörperteilen (zweites Grundkörperteil 11 und drittes Grundkörperteil 12). Das zweite Grundkörperteil 11 und das dritte Grundkörperteil 12 sind über jeweils ein Filmscharnier 13, 14 an das erste Grundkörperteil 10 angeschlossen und daher gelenkig um jeweils eine Scharnierachse des Filmscharniers 13, 14 relativ zu dem ersten Grundkörperteil 10 beweglich. Die beiden Grundkörperteile 11 und 12 sind identisch ausgeformt, aber relativ zu einer Symmetrieebene, die parallel zu den beiden Filmscharnieren 13, 14 angeordnet ist und durch die Längsachse 7 des Grundkörpers verläuft, symmetrisch angeordnet.

In einem Innenraum des Grundkörpers 2 ist ein im Wesentlichen hohlzylinderförmiger Gleitkörper 15 angeordnet und in axiale Richtung (d.h. die Richtung der Längsachse 7) beweglich, relativ zu dem Grundkörper 2, gelagert. In einen distalen Endabschnitt 16 des Gleitkörpers 2 ist eine Kanüle 17, die an ihrem distalen Ende mit einer durch eine Schlifffläche gebildeten Spitze 18 versehen ist, dichtend eingesetzt. In einen proximalen Endabschnitt 19, der als Muffenabschnitt ausgebildet ist, ist ein distaler Endabschnitt 20 des Schlauchs 5 dichtend eingesetzt. Aus diesem Grunde bilden der Gleitkörper 15, die Kanüle 17 und der Schlauch 5 eine fest miteinander verbundene Einheit, deren Bestandteile - abgesehen von der Flexibilität des Schlauchs 5 - nicht relativ zueinander verlagerbar sind. Um den Gleitkörper 15 in der Gebrauchsposition unter Vorspannung in dem Grundkörper 2 lagern und auf diese Weise einen Antrieb für eine proximale Richtung verlaufende Rückzugsbewegung des Gleitkörpers 15 relativ zu dem Grundkörper 2 bereitzustellen, ist auf den distalen Endabschnitt 16 des Gleitkörpers 15 äußerlich ein Stellelement 21 in Form einer Schraubenfeder aufgeschoben, die sich mit ihrem distalen Ende 22 in dem ersten Grundkörperteil 10 und mit ihrem proximalen Ende 23 an einem im Querschnitt rechteckförmigen Flansch 24, der über eine äußere Mantelfläche des Gleitkörpers 15 vorsteht, abstützt.

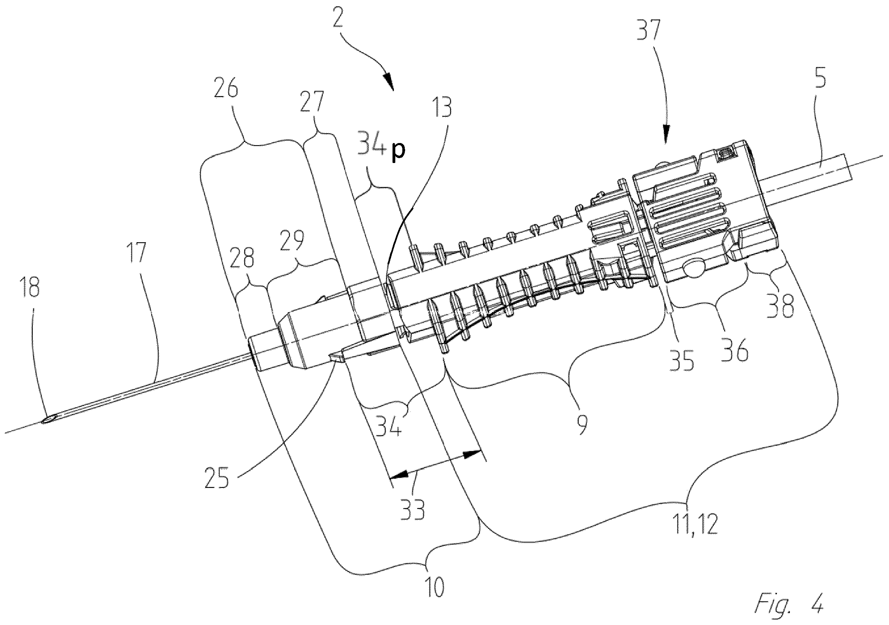

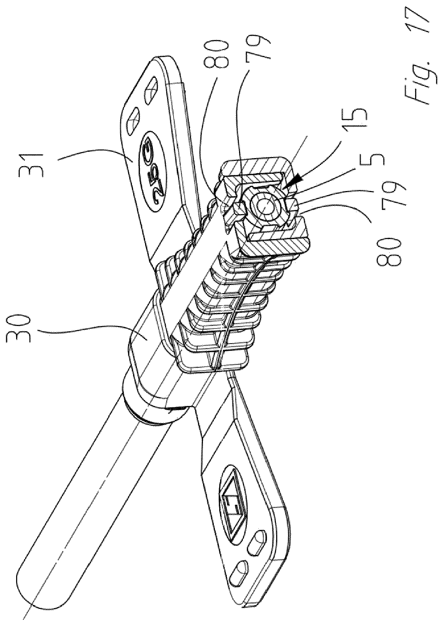

Im Folgenden wird anhand der Figuren 4 bis 10 der Aufbau des Grundkörpers 2 aus dessen Grundkörperteilen 10 bis 12 sowie das Zusammenwirken des Grundkörpers 2 mit dem Gleitkörper 15 näher erläutert:
Aus Figur 4 lässt sich erkennen, dass der Grundkörper 2, in axiale Richtung betrachtet, in verschiedene Bereiche unterteilt werden kann. Ausgehend von einem distalen Ende des Grundkörpers 2 befinden sich in dem hülsenförmigen ersten Grundkörperteil 10 zunächst ein rotationssymmetrischer (abgesehen von zwei Rastnasen 25 für die Fixierung des Flügelmoduls 26) und ein sich daran in proximale Richtung anschießender ungefähr quaderförmiger Abschnitt 27. Der rotationssymmetrische Abschnitt 26 ist seinerseits unterteilt in einen zylindrischen Spitzenabschnitt 28, auf den der in Figur 4 nicht dargestellte Kanülenschutz 4 (siehe Fig. 1 und 2) aufgeschoben wird, und einen zylindrischen, in seinem distalen Ende konsich abgeschrägten Übergangsabschnitt 29, in dem sich der hier nicht sichtbare distale Endabschnitt 16 des Gleitkörpers 15 samt dem darauf aufgeschobenen Stellelement 21 befindet. Wie sich mit Blick auf die Figuren 3 sowie 1 und 2 erkennen lässt, ist das Flügelmodul 3 mit einem mittleren Verbindungsteil 30, an dessen beiden gegenüberliegenden Längsseiten jeweils ein Griffflügel 31 einstückig angeschlossen ist (siehe auch Figur 17), von einem distalen Ende des Grundkörpers 2 her auf diesen aufgeschoben. Eine in axiale Richtung gemessene Länge 32 des Verbindungsteils 31 des Flügelmoduls 3 entspricht dabei einer Länge 33 eines Überlappungsbereichs 34, der sich sowohl über das erste Grundkörperteil 10 als auch die aneinander gefügten (zweiten und dritten) Grundkörperteile 11 und 12 erstreckt und in die Abschnitte 34d und 34p unterteilt werden kann, von denen der distale Abschnitt 34d das erste Grundkörperteil 10 und der proximale Abschnitt 34p das zweite und dritte Grundkörperteil 11, 12 überlappt. Im Bereich des ersten Grundkörperteils 10 enthält der Überlappungsbereich 34 vollständig den quaderförmigen Abschnitt 27 und teilweise den ungefähr zylindrischen Übergangsabschnitt 29 und zwar bis zu den dortigen Rastnasen 25. Das Verbindungsteil 30 des Flügelmoduls 3 überbrückt somit insbesondere auch den Bereich der beiden Filmscharniere 13, 14 und einen sich dort befindenden Spaltbereich zwischen dem ersten Grundkörperteil 10 und dem zweiten und dritten Grundkörperteil 11 und 12. Ausgehend von einem proximalen Ende des Überlappungsbereichs 34 schließt sich in proximale Richtung der bereits weiter oben beschriebene Griffbereich 9 an, der gemeinsam von beiden Grundkörperteilen 11 und 12 gebildet wird. Weiter in proximale Richtung befindet sich ein umlaufender Einkerbungsbereich 35 und weiter in proximale Richtung daran anschließend ein Auslösebereich 36, in dem sich ein später noch genauer erläuterter Auslösemechanismus 37 befindet. Ein proximaler Endabschnitt des Grundkörpers 2 wird schließlich von einem Verschlussbereich 38 gebildet, in dem das zweite Grundkörperteil 11 und das dritte Grundkörperteil 12 mittels Schnapphaken formschlüssig miteinander verbunden sind. Eine Öffnung des Grundkörpers 2 im Wege einer Klappbewegung der beiden Grundkörperteile 11 und 12 wird somit zum einen durch die Schnapphaken im Verschlussbereich 38 und zum anderen durch das übergeschobene Verbindungsteil 30 des Flügelmoduls 3 verhindert.

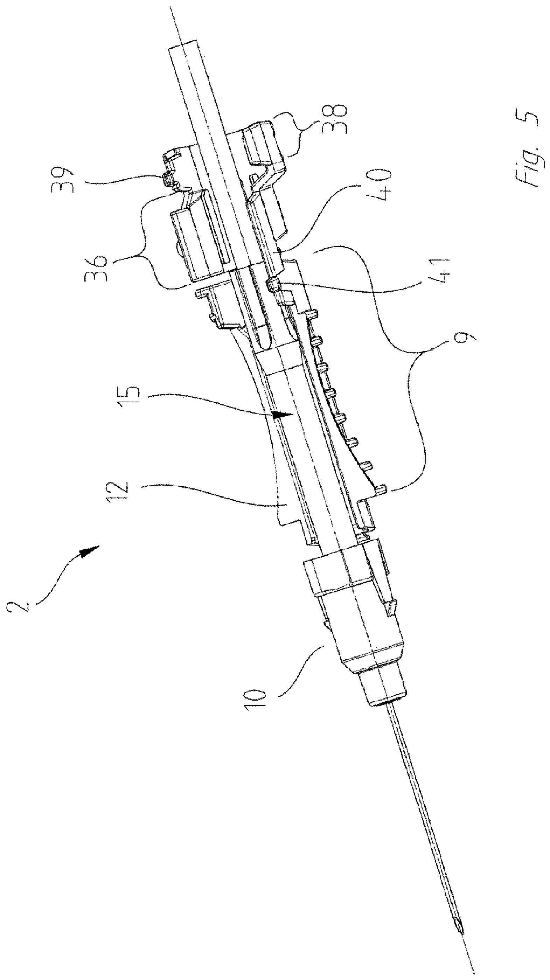

In Figur 5 ist gezeigt, wie der Gleitkörper 15 in dem Innenraum des Grundkörpers 2 angeordnet ist, wozu das zweite Grundkörperteil 11 nicht dargestellt ist, sondern nur das "untere" dritte Grundkörperteil 12. Der distale Endabschnitt 16 des Gleitkörpers 15 ist ebenso wie das Stellelement 21 nicht sichtbar, weil sie in dem hülsenförmigen ersten Grundkörperteil 10 verdeckt angeordnet sind. In dem Verschlussbereich 38 ist ein Schnapphaken 39 erkennbar, der mit einer angepassten Ausnehmung in dem zweiten Grundkörperteil 11 in zusammengefügtem Zustand der beiden Grundkörperteile 11 und 12 zusammenwirkt und einen nicht zerstörungsfrei lösbaren Verschluss bildet. Der Auslösebereich 36 und der Griffbereich 9 sind bei jedem der beiden Grundkörperteile 11, 12 ausschließlich über einen Verbindungssteg 40 miteinander verbunden, der an seinem proximalen Ende mit dem Griffbereich 9 und entsprechend an seinem distalen Ende mit dem Auslösebereich 36 verbunden ist. Es sei an dieser Stelle der Vollständigkeit halber vermerkt, dass das gesamte zweite und dritte Grundkörperteil 11, 12 (ebenso wie der aus den drei Grundkörperteilen 10, 11, 12 bestehende Grundkörper 2 insgesamt) einschließlich der Filmscharniere 13, 14 als einstückiges Spritzgussteil hergestellt wird. Figur 5 lässt darüber hinaus einen weiteren Schnapphaken 41 an dem Grundkörperteil 12 erkennen, wobei dieser Schnapphaken 41 im Griffbereich 9 angeordnet ist und ebenfalls mit einer angepassten Ausnehmung in dem zweiten Grundkörperteil 11 zusammenwirkt und die beiden Grundkörperteile 11 und 12 (zusätzlich zu den Schnapphaken 39 und dem Verbindungsteil 30 des Flügelmoduls 3) zusammenhält.

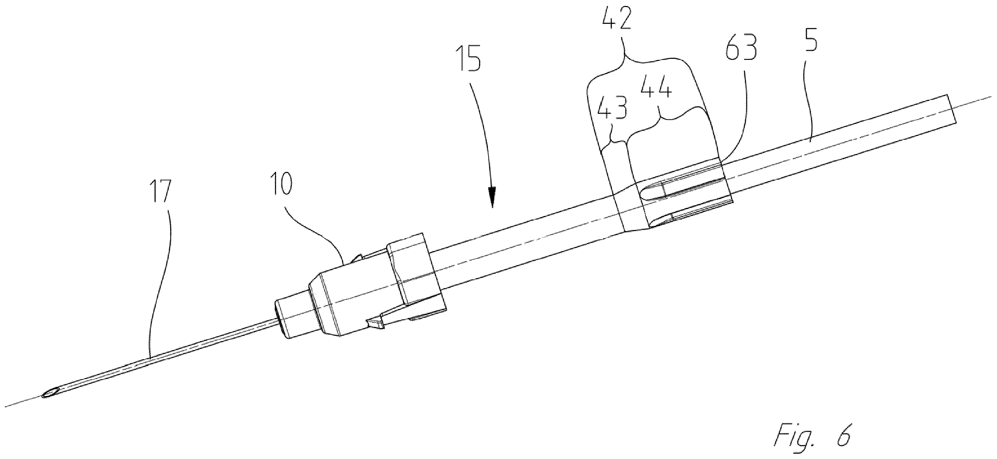

In Figur 6 ist der Gleitkörper 15 samt der darin eingesetzten Kanüle 17 und dem am proximalen Ende eingeschobenen Schlauch 5 dargestellt, wobei lediglich noch das erste Grundkörperteil 10 abgebildet ist. Der Gleitkörper 15 besitzt einen gegenüber einem Mittelabschnitt im Durchmesser vergrößerten und sich in proximale Richtung daran anschließenden Muffenabschnitt 42, der aus einem kürzeren Übergangsabschnitt 43 und einem sich in proximale Richtung daran anschließenden, von der Grundgestalt her zylindrischen Einsteckabschnitt 44 gebildet ist. Der Einsteckabschnitt 44 besitzt eine innere zylindrische Bohrung, die an einen Außendurchmesser des flexiblen Schlauchs 5 angepasst ist und in die der Schlauch dicht eingeklebt ist.

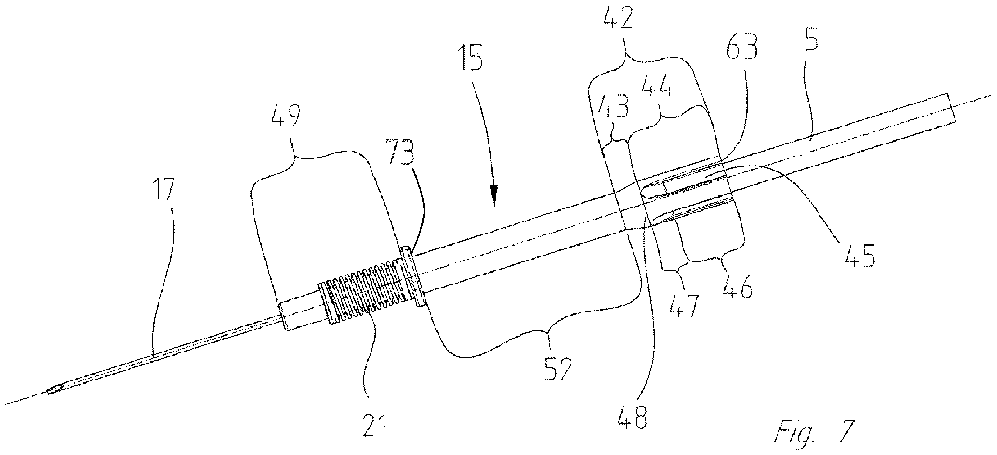

Mit Blick auf Figur 7 wird ersichtlich, dass der Muffenabschnitt 42 mit vier gleichmäßig über den Umfang des Muffenabschnitts 42 verteilt angeordneten und in Richtung der Längsachse des Gleitkörpers 15 verlaufenden Entspannungsnuten 45 für weiter unten erläuterte Blockierzungen des Grundkörpers 2 versehen ist. Die Entspannungsnuten 45 besitzen einen längeren Abschnitt 46, in dem sie eine größere Tiefe besitzen und einen parallel zu der Längsachse 7 verlaufenden Nutgrund, und einen kürzeren distalen Abschnitt 47, in dem der Nutgrund rampenförmig zu einer umlaufenden Grenzlinie 48 zwischen dem Einsteckbereich 44 und dem konischen Übergangsabschnitt 43 ansteigt. Die Funktion der Entspannungsnuten 45 in Verbindung mit den vorgenannten Blockierzungen wird später erläutert.

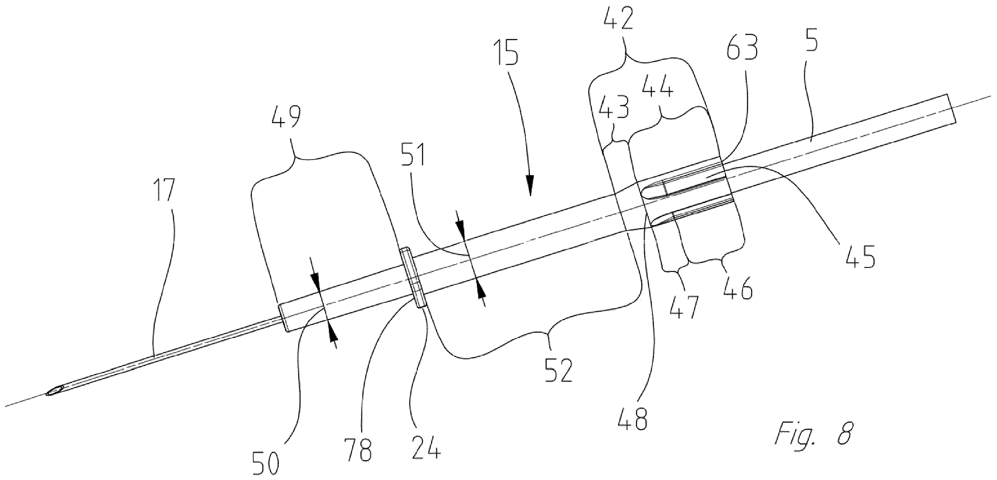

Während in Figur 7 auf einem distalen Endabschnitt 49 des Gleitkörpers 15 der Anschaulichkeit halber das komprimierte Stellelement 21 dargestellt ist, ist dies bei der im Übrigen analogen Darstellung gemäß Figur 8 nicht der Fall. Hierdurch wird deutlich, dass ein Durchmesser 50 in dem distalen Endabschnitt 49 des Gleitkörpers 15 größer ist als ein Durchmesser 51 in einem Mittelabschnitt 52 des Gleitkörpers 15.

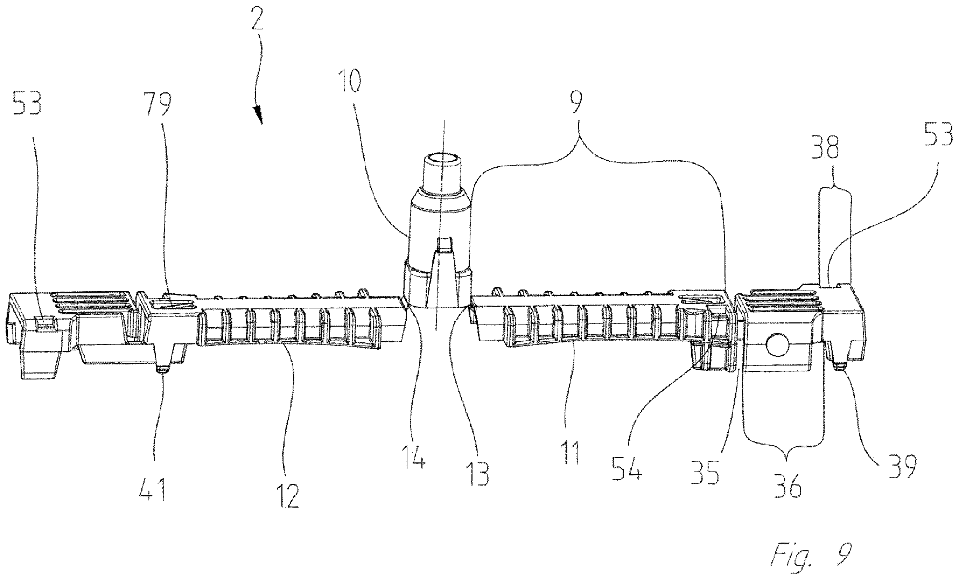

Die einzelnen Bereiche des Grundkörpers 2 und die spiegelsymmetrische Anordnung der identisch ausgeformten Grundkörperteile 11 und 12, lässt sich sehr anschaulich der Figur 9 entnehmen, in der der Grundkörper 2 mit von dem ersten Grundkörperteil 10 seitlich abgeklappten Grundkörperteilen 11 und 12 dargestellt ist. In diesem Zustand wird der Grundkörper 2 als Spritzgussteil aus dem Spritzgusswerkzeug entnommen. Anschaulich lassen sich aus der Figur 9 der Griffbereich 9, der sich anschließende Einkerbungsbereich 35, der sich daran anschließende Auslösebereich 36 und der den proximalen Abschluss bildende Verschlussbereich 38 entnehmen. Auf der in Figur 9 links dargestellten dritten Grundkörperteil 12 ist eine Ausnehmung 53 erkennbar, in der der Schnapphakten 39 (s. Fig. 5) verriegelnd aufnehmbar ist. Auch eine weitere Ausnehmung 54 in dem Griffbereich 9 ist erkennbar, die zur verriegelnden Aufnahme des korrespondierenden Schnapphakens 41 am Griffbereich 9 des dritten Grundköperteils 12 dient. Somit existieren insgesamt zwei Schnapphaken 39 und zwei Ausnehmungen 53, in dem Verschlussbereich 38 und zwei Schnapphaken 41 und zwei Ausnehmungen 54 in dem Griffbereich 9. An jedem der beiden Grundkörperteile 11 und 12 ist somit sowohl in dem Griffbereich 9 als auch in dem Verschlussbereich 38 jeweils ein Schnapphaken 39, 41 und jeweils eine Ausnehmung 53, 54 vorhanden.

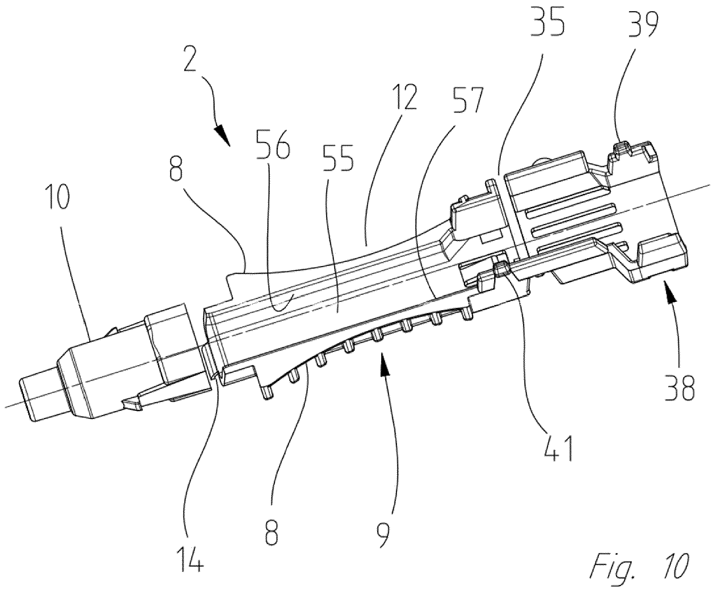

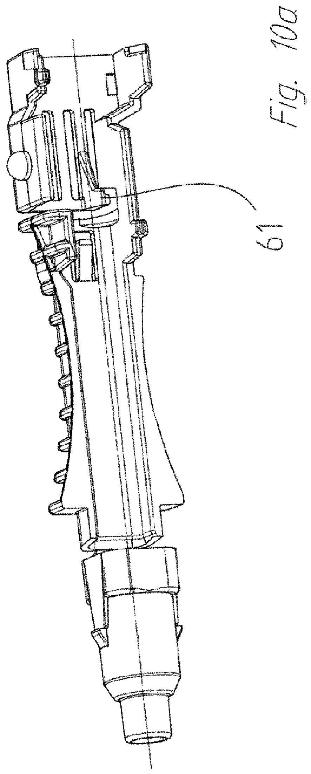

Figur 10 lässt einen Einblick in das rinnenförmig gestaltete dritte Grundkörperteil 12 zu, da einerseits das im gefügten Zustand darüber angeordnete ebenfalls rinnenförmige zweite Grundkörperteil 11 entfernt wurde und andererseits auch der Gleitkörper 15 nebst Kanüle 17 und Schlauch 5 nicht eingelegt ist. Es lässt sich erkennen, dass ein Boden 55 und zwei gegenüberliegende Halbwandungen 56, 57 des Grundkörperteils 12 im rechten Winkel zueinander angeordnet sind, wodurch sich im gefügten Zustand der beiden Grundkörperteile 11, 12 ein quadratischer Querschnitt des Innenraums ergibt. Die beiden auf gegenüberliegenden Seiten angeordneten Längsrippen 8 des Grundkörperteils 12 werden von beiden Grundkörperteilen 11 und 12 im gefügten Zustand gemeinsam ausgebildet und definieren im Kontaktbereich der beiden Grundkörperteile 11, 12 eine parallel zu der Längsachse 7 verlaufende Mittelebene, die auch eine Symmetrieebene bildet. Außerdem sind in Figur 10 auch die auf gegenüberliegenden Seiten - in Bezug auf die Längsachse 7 - angeordneten Schnapphaken 41 im Griffbereich 9 und 39 im Verschlussbereich 38 erkennbar.

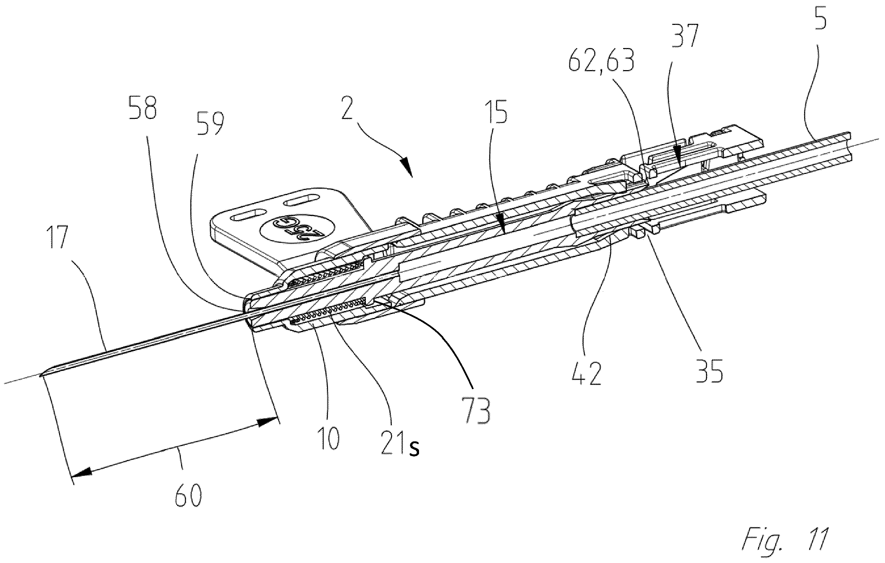

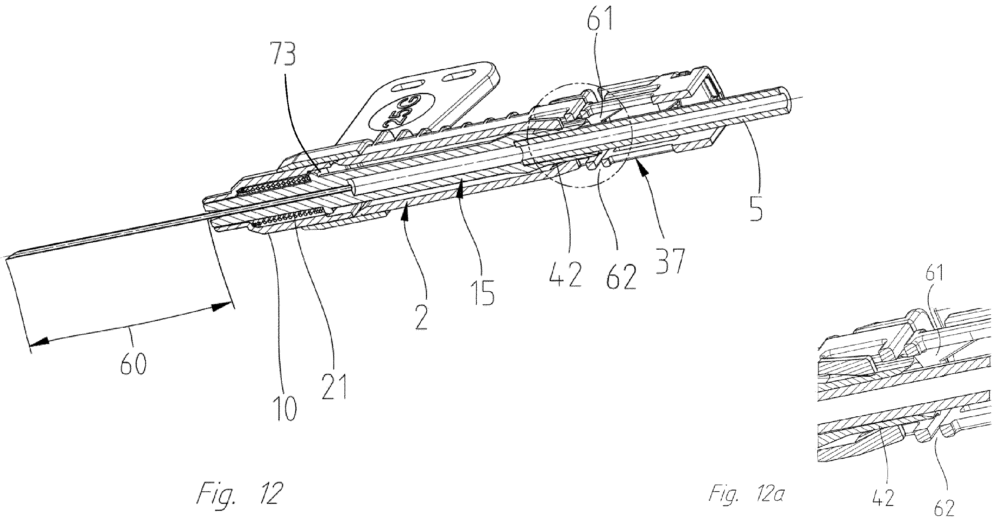

In den Figuren 11, 12 und 12a ist aus unterschiedlichen Perspektiven jeweils ein Längsschnitt der Sicherheitskanülenanordnung 1 im Gebrauchszustand des Gleitkörpers 15 dargestellt. Eine distale Stirnfläche 58 des Gleitkörpers 15 schließt dabei im Wesentlichen bündig mit einer distalen Stirnfläche 59 des ersten Grundkörperteils ab. Die Kanüle 17 steht in dieser Stellung mit einer freien Länge 60 über die distale Stirnfläche 59 des Grundkörperteils 10 vor. Das Stellelement 21 befindet sich in einem vorgespannten Zustand, so dass der Gleitkörper 15 bestrebt ist, sich relativ zu dem Grundkörper 2 in proximale Richtung zu bewegen. Diese Bewegung wird durch zwei Verriegelungselemente 61a, 61b unterbunden, die Teil des Auslösemechanismus 37 sind und mit einer proximalen Stirnfläche 62 des Gleitkörpers 15 in dessen Muffenabschnitt 42 zusammenwirken. Die Stirnfläche 62 des Gleitkörpers 15 bildet in Verbindung mit dem im Durchmesser kleineren Schlauch 5 eine Stufe 63 aus, die auch in den Figuren 6 bis 8 gut erkennbar ist. Diese Stufe befindet sich in der Gebrauchsstellung des Gleitkörpers 15 - in axiale Richtung betrachtet - in dem Einkerbungsbereich 35 und zwar an dessen proximalen Ende, das durch die Verriegelungselemente 61a, 61b des Auslösemechnanismus 37 definiert wird. Der Gleitkörper 15 ist somit in der Gebrauchsstellung in axiale Richtung in dem Grundkörper 2 spielfrei festgelegt, was für einen ordnungsgemäßen Punktionsvorgang unerlässlich ist.

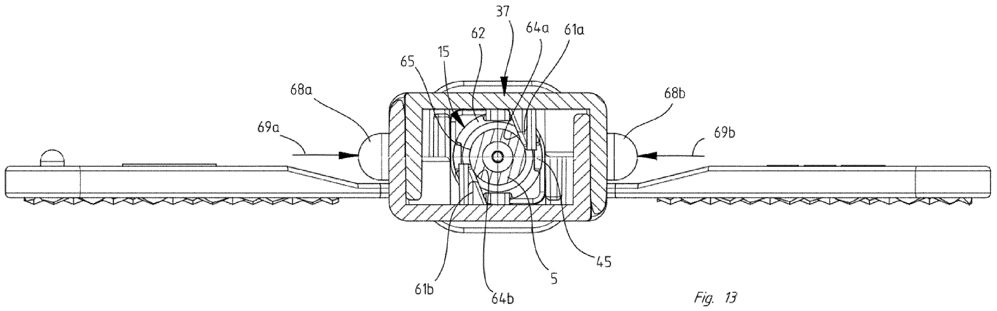

Aus einer Zusammenschau der Figuren 13 bis 16 sowie 18 wird die Funktionsweise des Auslösemechnanismus 37 deutlich. Die proximale Stirnfläche 62, die die besagte Stufe 63 im Übergang von dem Schlauch 5 zu dem Muffenabschnitt 42 des Gleitkörpers 15 bildet, wird von zwei diametral gegenüberliegenden Verriegelungselementen 61a, 61b (siehe auch Fig. 10 a) zurückgehalten, so dass der Gleitkörper 15 in der Gebrauchsstellung 45 verharrt. In dem Muffenabschnitt 42 des Gleitkörpers 15 sind die Entspannungsnuten sichtbar, woraus sich im Querschnitt eine durch vier Vertiefungen im 90° Grad-Abstand unterbrochene Kreiskontur der Stirnfläche 62 ergibt.

Die Verriegelungselemente 61a, 61b besitzen - bei axialer Blickrichtung - eine Dreiecks- bzw. Trapezform und liegen mit jeweils einer Steuerkante 64a, 64b an einer äußeren Mantelfläche 65 des Schlauchs 5 an.

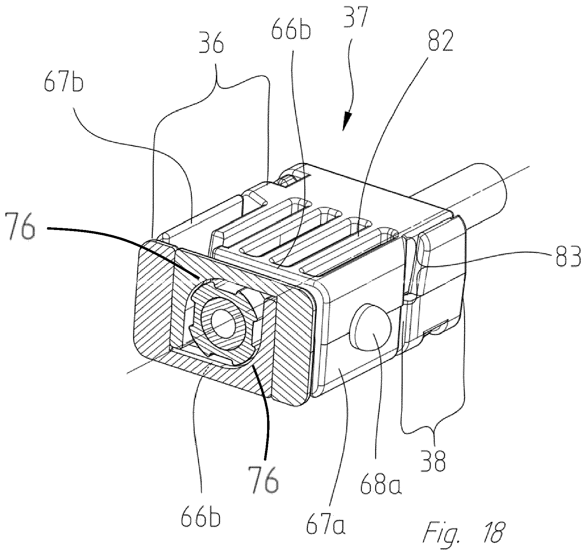

Aus Figur 18 lässt sich entnehmen, dass die Verriegelungselemente 61a, 61b über jeweils einen Druckstab 66a, 66b mit einem Auslöseglied 67a, 67b gekoppelt sind. Die Auslöseglieder 67a, 67b sind jeweils mit einer Erhebung 68a, 68b in Form eines halbkugelförmigen Knopfes versehen. Die Auslöseglieder 67a, 67b bilden in einer Seitenansicht des Grundkörpers 2 zwei ungefähr quadratische Flächen jeweils mit der zentralen Erhebung 68a, 68b, wobei die Druckstäbe 66a, 66b hierzu in einem Winkel von 90° Grad verlaufen. Die Verriegelungselemente 61a, 61b erstrecken sich wiederum in einem Winkel von ungefähr 90° Grad zu den Druckstäben 66a, 66b, so dass sich insgesamt eine C-Form bzw. U-Form ergibt. Dabei bilden die Auslöseglieder 67a, 67b und die Verriegelungselemente 61a, 61b jeweils einen freien Schenkels des U bzw. C, hingegen die Druckstäbe 66a, 66b ein "Basiselement" des U bzw. C.

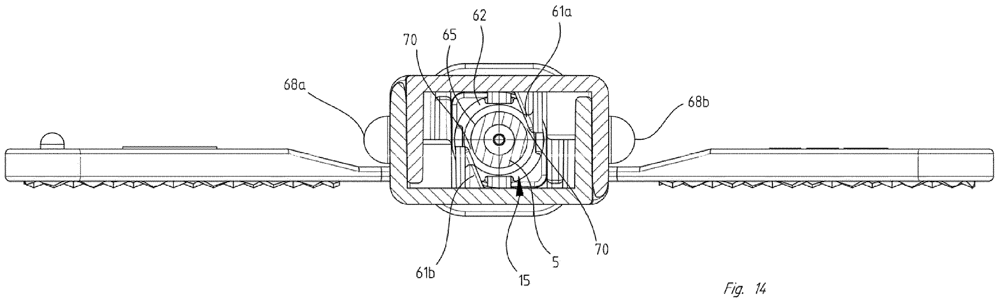

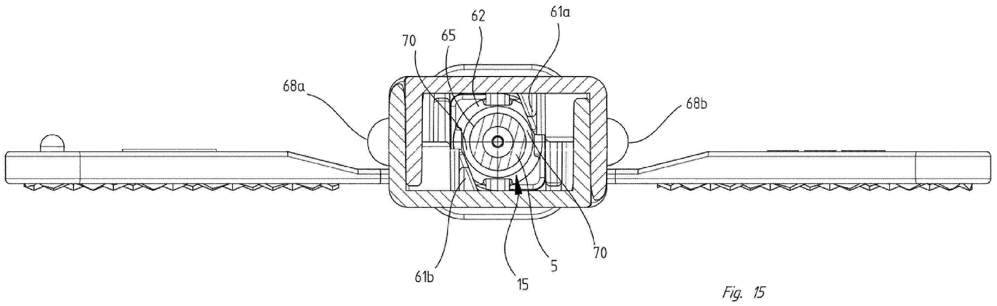

Soll nach Beendigung der mittels der Sicherheitskanülenanordnung 1 durchgeführten Blutentnahme oder Infusion der Gleitkörper 15 in die Sicherungsstellung überführt werden, in der die Kanüle 17 vollständig innerhalb des Grundkörpers 2 untergebracht ist, so übt der Anwender der Sicherheitskanülenanordnung 1 ein mit zwei Fingern einer Hand gleichzeitig eine radial in Richtung der Pfeile 69a, 69b verlaufende Druckkraft auf die Erhebungen 68a, 68b der Auslöseglieder 67a, 67b aus. Ausgehend von der in Figur 13 dargestellten Verriegelungsstellung, in der die Verriegelungselemente 61a, 61b den Gleitkörper 15 durch einen Eingriff (Anschlag an die Stirnfläche 62) blockieren, bewegt sich das in Figur 13 obere Verriegelungselement 61a durch Druck auf das linke Auslöseglied 68a nach rechts, wohingegen sich das in Figur 13 untere Verriegelungselement 61b durch Druck auf das rechte Auslöseglied 68b nach links bewegt. In Figur 14 ist ein Zustand gezeigt, in dem sich durch entsprechende Verlagerung der eine starre Einheit bildenden Auslöseglieder 68a, 68b und den damit verbundenen Verriegelungselementen 61a, 61b zwischen letzteren und der äußeren Mantelfläche 65 des Schlauchs 5 ein Abstand 70 gebildet hat. Dieser Abstand 70 ist allerdings noch nicht so groß, um einen Anschlag der Stirnfläche 62 an den Verriegelungselementen 61a, 61b aufzuheben. Aus diesem Grunde verharrt der Kanülenträger 15 in dieser Stellung des Auslösemechanismus bzw. der Auslöseglieder 67a, 67b immer noch in der Gebrauchsstellung. Figur 15 zeigt einen weiteren Zwischenzustand, in dem die Abstände 70 zwischen den Verriegelungselementen 61a, 61b und der Mantelfläche 65 des Schlauchs 5 zwar größer sind, gleichwohl aber immer noch eine Überlappung zwischen den Verriegelungselementen 61a, 61b und der Stirnfläche 62 des Gleitkörpers 15 und damit dessen Blockade bewirken.

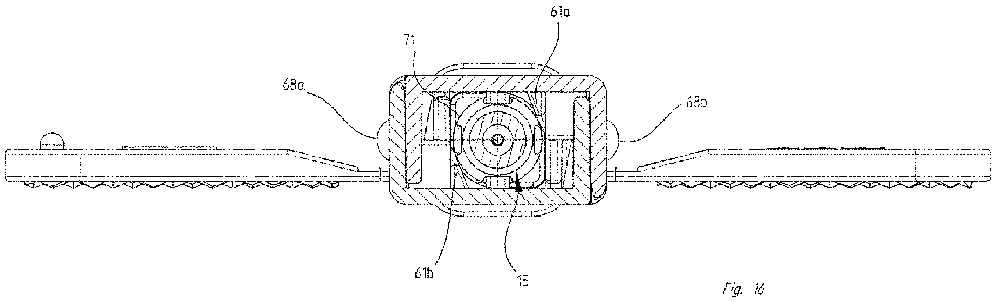

In der in Figur 16 gezeigten Position der Auslöseglieder 67a, 67b mit ihren zugehörigen knopfförmigen Erhebungen 68a, 68b sind die zugeordneten Verriegelungselemente 61a, 61b gerade soweit nach außen verlagert, dass der durch eine kreisförmige Umrisslinie 71 (Einhüllende) definierte Querschnitt des Gleitkörpers 15 im Bereich des Muffenabschnitts 42 vollständig freigegeben ist. Nunmehr sind die Verriegelungselemente 61a, 61b außer Eingriff mit dem Gleitkörper 15, so dass sich letzterer, angetrieben durch das Stellelement 21, in proximale Richtung bewegen kann, was zu einem Rückzug der Kanüle 17 in das Innere des Grundkörpers 2 führt.

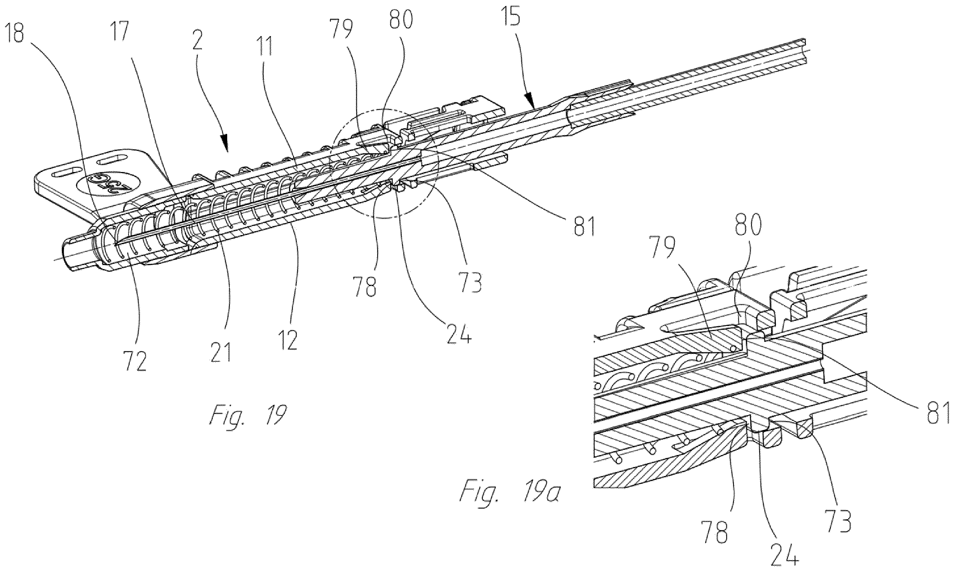

Dementsprechend zeigen die Figuren 19 und 19a den Gleitkörper 15 in der Sicherungsstellung, in der die Kanüle 17 einschließlich ihrer Spitze 18 innerhalb eines Innenraums 72 des Grundkörpers 2 angeordnet ist. Das Stellelement 21 in Form der Schraubenfeder liegt nunmehr in einem gegenüber der Gebrauchsstellung des Gleitkörpers 15 entspannteren Zustand vor.

Um einen Austritt des gesamten Gleitkörpers 15 und damit auch der Kanüle 17 selbst an dem proximalen Ende des Grundkörpers zu verhindern, wird die Sicherungsstellung des Gleitkörpers 15 durch eine an diesem angeordnete Anschlagfläche 73 definiert. Die am Gleitkörper 15 befindliche Anschlagfläche 73 wirkt mit einer Anschlagfläche 74 des Grundkörpers 2 zusammen, die sich am proximalen Ende des Grundkörpers 2 und zwar am proximalen Ende des Verschlussbereichs 38, der gemeinsam von dem zweiten Grundkörperteil 11 und dem dritten Grundkörperteil 12 gebildet wird, ausgeformt ist.

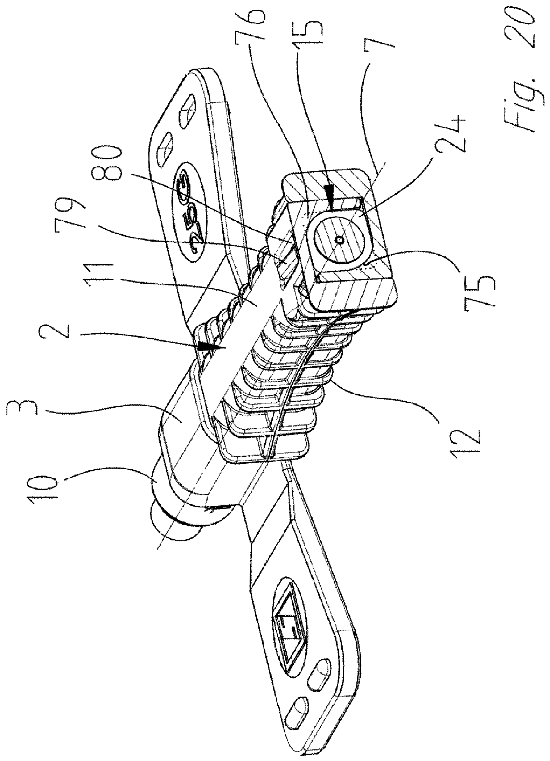

Wie sich aus der Schnittdarstellung gemäß Figur 20 ergibt, besitzt sowohl das zweite als auch das dritte Grundkörperteil 11, 12 im Querschnitt eine U-förmige Gestalt, wobei die beiden U-förmigen Querschnitte der Wandungen in eine Richtung senkrecht zu der Symmetrieebene des Grundkörpers 2, d.h. in eine Richtung senkrecht zu den beiden Filmscharnieren, d.h. der Verschwenkung beim Fügevorgang, ineinander verschachtelt sind und somit einen im Wesentlichen quadratischen freien Querschnitt im Innenraum 72 des Grundkörpers 2 definieren. Die Querschnittform im Bereich der Anschlagflächen 74 der Grundkörperteile 11 und 12 ist allerdings nicht exakt quadratisch sondern zwei Eckbereiche sind ausgefüllt und zu der Längsachse 7 des Grundkörpers 2 hin von jeweils einer Bogenlinie 75, die in die betreffende Ecke des U oder C einen Viertelkreis einbeschreibt. In Figur 20 sind die Kanten eines gedachten Quadrats, in das in den Ecken die durch die Bogenlinien 75 begrenzten Anschlagelemente 76 eingebracht sind, der besseren Anschaulichkeit halber gestrichelt dargestellt.

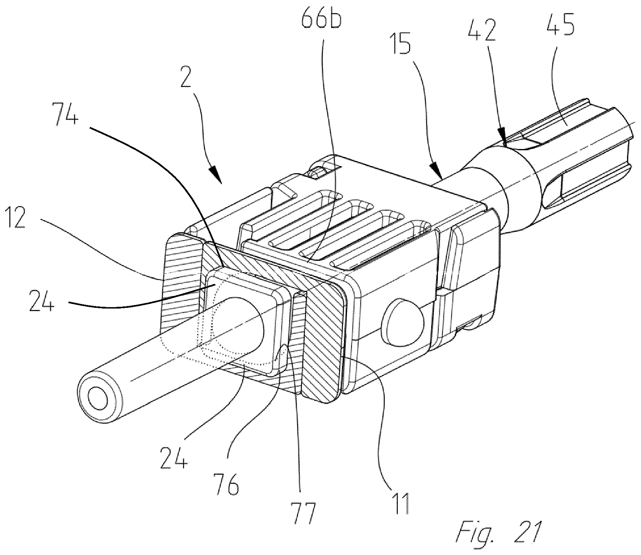

Wie aus Figur 20 zu erkennen ist, ist der Radius eines Kreises, der bei einem Blick in Richtung der Längsachse 7 durch die (leicht abgerundeten) Ecken des quadratförmigen Flansches 24 verläuft, größer als der Radius eines Kreises, der durch eine Fortsetzung der beiden Bogenlinien 75 gebildet wird. Aus diesem Grunde kann der quadratförmige Flansch 24 nicht durch den freien Querschnitt im Bereich der Anschlagflächen 74, die an den Anschlagelementen 76 ausgebildet sind, hindurchtreten, so dass eine Verlagerung des Gleitkörpers 15 an dieser Stelle beendet wird. Aus der Schnittdarstellung gemäß Figur 21 lässt sich dieser Sachverhalt ebenfalls gut entnehmen. Der quadratförmige Flansch 24 liegt im Bereich seiner abgerundeten Abschnitte 77 an den Anschlagflächen 76 der Grundkörperteile 11, 12 an. In dem in Figur 21 dargestellten Zustand befindet sich der Kanülenträger 15 in der Sicherungsstellung, in der der Muffenabschnitt 42 am proximalen Ende des Gleitkörpers 15 aus dem Grundkörper 2 aus diesem ausgetreten ist.

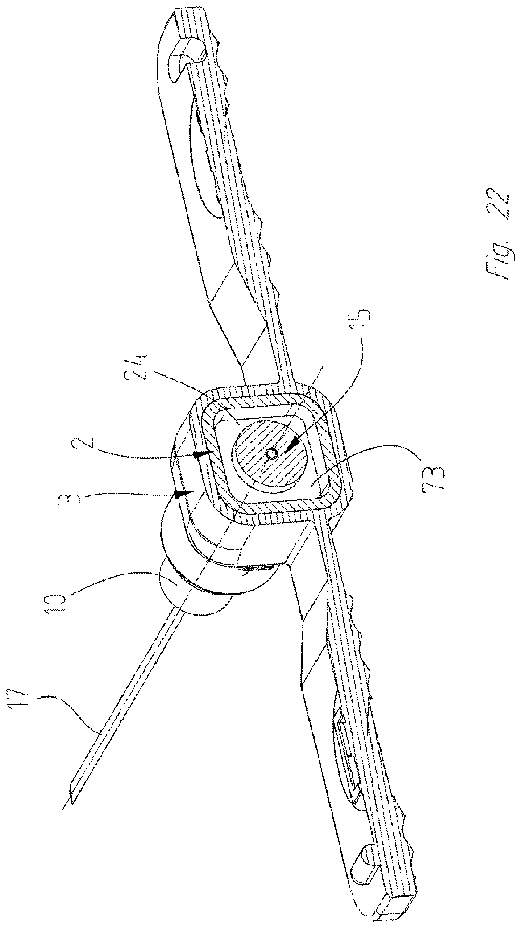

Aus Figur 22 lässt sich erkennen, dass der Innenraum 72 in dem quaderförmigen Abschnitt 27 des insgesamt hülsenförmigen ersten Grundkörperteils 10 quadratisch (mit abgerundeten Ecken) ist. An diese Querschnittsform ist der Querschnitt des quadratförmigen Flansches 24 so angepasst, dass einerseits eine leicht gängige Bewegung in axiale Richtung des Gleitkörpers 15 unter Einwirkung der Kraft des Stellelements 21 möglich ist, andererseits jedoch eine Drehung des Gleitkörpers 15 um die Längsachse 7 sicher unterbunden wird. Der Flansch 24 erfüllt somit die Funktion eines Drehblockierkörpers des Gleitkörpers 15 und entsprechend der quaderförmige Abschnitt 27 des ersten Grundkörperteils 10 einen Drehblockierkörper des Grundkörpers 2. Während an dem quadratförmigen Flansch 24 des Gleitkörpers 15 die proximale Anschlagfläche 73 zur Begrenzung der Rückzugsbewegung des Gleitkörpers 15 angeordnet ist, besitzt der quadratförmige Flansch 24 insofern eine Doppelfunktion, als seine in Figur 22 nicht sichtbare, allerdings in den Figuren 7 und 8 veranschaulichte weitere Anschlagfläche 78 zur Abstützung des Stellelements 21 dient.

Neben einer Begrenzung der Rückzugsbewegung des Gleitkörpers 15 in proximale Richtung, ist es außerdem wichtig zu verhindern, dass der Gleitkörper 15, nachdem er einmal die Sicherungsstellung eingenommen hat, absichtlich oder unabsichtlich wieder in distale Richtung vorgeschoben wird, um die Kanüle 17 wieder freizulegen und die Sicherheitskanülenanordnung 1 möglicherweise wiederzuverwenden, was unbedingt vermieden bzw. verhindert werden soll. Zu diesem Zweck dienen die bereits weiter oben angesprochenen Blockierzungen 79, die als federnde Elemente des Grundkörpers 2 ausgebildet und jeweils innerhalb eines die Blockierzungen 79 dreiseitig umgebenden Fensters 80 angeordnet sind. Dieser Umstand lässt sich den Figuren 19 und 19a gut entnehmen, in denen sich der Gleitkörper 15 in der Sicherungsstellung befindet. Jeweils eine Stirnfläche 81 der Blockierzungen 79 liegt an der in distale Richtung orientierten Anschlagfläche 78 des quadratförmigen Flansches 24 an, so dass diesem sogar eine dreifache Funktionalität zukommt (Endanschlag der Rückzugsbewegung, Abstützung des Stellelements 21 sowie Anschlagfläche für die Blockierzungen 79). Da die Blockierzungen 79 elastisch mit dem jeweiligen Grundkörperteil 11, 12 verbunden sind, weichen sie beim Hindurchlaufen des Gleitkörpers 15 während der Rückzugsbewegung elastisch radial nach außen aus, um sich nach Passage des quadratförmigen Flanschs 24 durch ihre Vorspannung wieder radial nach innen zu verlagern und die Blockierwirkung in Verbindung mit dem Flansch 24 zu erzielen. Da der Durchmesser des Gleitkörpers 15 in dem distalen Endabschnitt 49 kleiner ist als in dem Mittelbereich 52, ist eine hinreichend große Überlappung der Blockierzungen 79 mit dem Vorsprung des Flansches 24 über die angrenzende Zylinderfläche (in radiale Richtung betrachtet) sichergestellt.

Wie sich aus Figur 9 entnehmen lässt, werden die Blockierzungen 79 bei der spritzgusstechnischen Herstellung der Grundkörperteile 11 und 12 so ausgeformt, dass sie, ausgehend von einem Verbindungsquerschnitt mit dem zugeordneten Grundkörperteil 11, 12 hin zu ihren freien Enden, auf die Längsachse 7 des Grundkörper 2 bzw. Gleitkörpers 15 zu geneigt verlaufen. Um diese Vorspannung radial nach innen sicher auch während einer möglicherweise längeren Lagerzeit der Sicherheitskanülenanordnung 1 aufrecht zu erhalten, sind in dem Muffenbereich 42 des Gleitkörpers 15 die bereits zuvor erläuterten Entspannungsnuten 45 eingeformt. Diese erlauben während der Lagerung im Gebrauchszustand des Gleitkörpers 15 eine auf die Längsachse 7 zu geneigte Ausrichtung der Blockierzungen 79 genau in der Form, wie sie später in der Sicherungsstellung des Gleitkörpers 15 zur Blockierung einer erneuten Ausfahrbewegung des Gleitkörpers 15 benötigt wird. Auf diese Weise wird im Vergleich mit einer Gestaltung des Muffenabschnitts 42 ohne derartige Entspannungsnuten 45 eine Materialermüdung und ein Verlust der radial nach innen gerichteten Vorspannung verhindert, was ansonsten zu einem Versagen der Blockierzungen 79 führen könnte, so dass dann ein erneutes Ausfahren der Kanüle 17 nicht sicher verhindert wäre.

Bezüglich der Ausgestaltung des Auslösemechanismus 37 sei im Hinblick auf Figur 18 sowie ergänzend auch die Figuren 10, 10 a und 4 noch Folgendes ausgeführt:
Wie bereits erwähnt, ist der Grundkörper 2 im Auslösebereich 36 ungefähr quaderförmig, wobei jeweils ein Auslöseglied 67a, 67b und der zugeordnete Druckstab 66a, 66b in Verbindung mit als Balken 82 ausgebildeten Gelenkelementen, von denen drei durch den jeweiligen Druckstab 66a, 66b verbunden werden, gemeinsam eine L-förmige Wandanordnung bilden. Aufgrund der geringen Querschnitte der Balken 82, lassen sich diese durch Druck auf die Auslöseglieder 67a relativ zu dem proximalen Verschlussbereich 38 verformen. Bei Druck auf die Auslöseglieder 67a, 67b wirkt darüber hinaus ein zwischen diesen und dem Verschlussbereich 38 angeordneter Verbindungsquerschnitt 83 als weiteres (Dreh-) Gelenk. Eine von den Auslösegliedern 67a, 67b jeweils gebildete Auslösefläche verläuft erkennbar unter einem Winkel von 90° Grad zu einer Ebene, innerhalb der die Balken 82 und der diese jeweils verbindende Druckstab 66a, 66b angeordnet sind. Die jeweiligen Verriegelungselemente 61a, 61b erstrecken sich wiederum unter einem Winkel von 90° Grad zu letztgenannter Ebene der Balken 82 sowie des jeweils zugeordneten Druckstabs 66a, 66b. Insgesamt ergibt sich im Querschnitt somit eine U- bzw. C-Anordnung.

### Bezugszeichenliste

- 1: Sicherheitskanülenanordnung
- 2: Grundkörper
- 3: Flügelmodul
- 4: Kanülenschutz
- 5: Schlauch
- 6: Querrippe
- 7: Längsachse
- 8: Längsrippe
- 9: Griffbereich
- 10: erstes Grundkörperteil
- 11: zweites Grundkörperteil
- 12: drittes Grundkörperteil
- 13: Filmscharnier
- 14: Filmscharnier
- 15: Gleitkörper
- 15m: Mantelfläche
- 16: distaler Endabschnitt
- 17: Kanüle
- 18: Spitze
- 19: proximaler Endabschnitt
- 20: distaler Endabschnitt
- 21: Stellelement
- 21s: Stützfläche
- 22: distales Ende
- 23: proximales Ende
- 24: Flansch
- 25: Rastnase
- 26: rotationssymmetrischer Abschnitt
- 27: quaderförmiger Abschnitt
- 28: Spitzenabschnitt
- 29: Übergangsabschnitt
- 30: Verbindungsteil
- 31: Griffflügel
- 32: Länge
- 33: Länge
- 34: Überlappungsbereich
- 34d: Abschnitt
- 34p: Abschnitt
- 35: Einkerbungsbereich
- 36: Auslösebereich
- 37: Auslösemechanismus
- 38: Verschlussbereich
- 39: Schnapphaken
- 40: Verbindungssteg
- 41: Schnapphaken
- 42: Muffenabschnitt
- 43: Übergangsabschnitt
- 44: Einsteckabschnitt
- 45: Entspannungsnut
- 46: Abschnitt
- 47: Abschnitt
- 48: Grenzlinie
- 49: Endabschnitt
- 50: Durchmesser
- 51: Durchmesser
- 52: Mittelbereich
- 53: Ausnehmung
- 54: Ausnehmung
- 55: Boden
- 56: Halbwandung
- 57: Halbwandung
- 58: Stirnfläche
- 59: Stirnfläche
- 60: freie Länge
- 61a, 61b: Verriegelungselement
- 62: proximale Stirnfläche
- 63: Stufe
- 64a, 64b: Steuerkante
- 65: Mantelfläche
- 66a, 66b: Druckstab
- 67a, 67b: Auslöseglied
- 68a, 68b: Erhebung
- 69a, 69b: Pfeil
- 70: Abstand
- 71: Umrisslinie
- 72: Innenraum
- 73: Anschlagfläche
- 74: Anschlagfläche
- 75: Bogenlinie
- 76: Anschlagelement
- 77: Abschnitt
- 78: Anschlagfläche
- 79: Blockierzunge
- 80: Fenster
- 81: Stirnfläche
- 82: Balken
- 83: Verbindungsquerschnitt
- 84: Drehblockiereinrichtung

## Patentansprüche

1. Sicherheitskanülenanordnung (1) umfassend
a) eine Kanüle (17) zur Punktion menschlichen oder tierischen Gewebes, wobei die Kanüle in einem distalen Endabschnitt eine Spitze (18) aufweist,
b) einen Gleitkörper (15), der in einem distalen Endabschnitt (49) mit der Kanüle (17) und in einem proximalen Endabschnitt (46) mit einem flexiblen Schlauch (5) versehen ist, wobei eine sich durch den Gleitkörper (15) erstreckende Strömungsverbindung zwischen der Spitze (18) der Kanüle (17) und einem proximalen Ende des Schlauchs (5) besteht,
c) einen Grundkörper (2), in dem der Gleitkörper (15) von einer Gebrauchsstellung, in der sich die Spitze (18) der Kanüle (17) außerhalb des Grundkörpers (2) befindet, in eine Sicherungsstellung verlagerbar ist, in der sich die Spitze (18) der Kanüle (17) innerhalb des Grundkörpers (2) befindet, wobei der Grundkörper (2) aus mindestens drei Grundkörperteilen (10, 11, 12) zusammengesetzt ist, von denen
- ein erstes Grundkörperteil (10) hülsenförmig ausgebildet ist und einen distalen Endabschnitt (49) des Gleitkörpers (15) in dessen Gebrauchsstellung umschließt,
- ein zweites Grundkörperteil (11) sich in proximale Richtung an das erste Grundkörperteil (10) anschließt und mit diesem verbunden ist, wobei das zweite Grundkörperteil (11) in einem Querschnitt U-förmig, L-förmig, C-förmig oder deckelförmig ausgebildet ist und
- ein drittes Grundkörperteil (12) und das zweite Grundkörperteil (11) zu einem an beiden gegenüberliegenden Stirnseiten offenen Hohlkörper zusammenfügbar sind, wobei das dritte Grundkörperteil (12) deckelförmig, C-förmig, L-förmig oder U-förmig ausgebildet und über ein Filmscharnier (13, 14) mit dem zweiten Grundkörperteil (11) oder dem ersten Grundkörperteil (10) verbunden ist.
d) ein zwischen dem Grundkörper (2) und dem Gleitkörper (15) angeordnetes Stellelement (21), mittels dessen der Gleitkörper (15) von der Gebrauchsstellung in die Sicherungsstellung verlagerbar ist und
e) ein an dem Grundkörper (2) angeordneten Auslösemechanismus (37), mittels dessen die Verlagerung des Gleitkörpers (15) von der Gebrauchsstellung in die Sicherungsstellung auslösbar ist, wobei der Auslösemechanismus (37) mindestens ein Auslöseglied (67a, 67b) aufweist, das von einer die Sicherheitskanülenanordnung (1) gebrauchenden Person mit einer radial gerichteten Druckkraft oder mit einem Drehmoment beaufschlagbar ist und das mit einem Verriegelungselement (61a, 61b) versehen ist, das infolge einer Beaufschlagung mit der Druckkraft oder dem Drehmoment von einer Verriegelungsstellung, in der es mit dem Gleitkörper (15) in Eingriff ist und diesen in der Gebrauchsstellung verriegelt, in eine Auslösestellung verlagerbar ist, in dem es außer Eingriff mit dem Gleitkörper (15) ist, sodass sich dieser in die Sicherungsstellung begibt,
**dadurch gekennzeichnet, dass** sich das Stellelement (21) mit seinem distalen Ende an einer Stützfläche (21s) des ersten Grundkörperteils (10) abstützt.

2. Sicherheitskanülenanordnung (1) nach Anspruch 1, **dadurch gekennzeichnet, dass** der Grundkörper (2) an zwei gegenüber liegenden Seiten mit jeweils einem Griffflügel (31) versehen ist.

3. Sicherheitskanülenanordnung (1) nach Anspruch 1 oder 2, **dadurch gekennzeichnet, dass das** Stellelement (21) sich mit seinem distalen Ende an einer in einem Innenraum des ersten Grundkörperteils (10) ausgebildeten Stufe oder einer einen distalen Abschluss des ersten Grundkörperteils (10) bildenden Stirnwand abstützt.

4. Sicherheitskanülenanordnung (1) nach einem der Ansprüche 1 bis 3, **dadurch gekennzeichnet, dass** die Griffflügel (31) an ein hülsenförmiges Verbindungsteil (30) angeschlossen sind, das auf den Grundkörper (2) aufgeschoben ist, wobei vorzugsweise ein Überlappungsbereich (34) zwischen dem Verbindungsteil (30) und dem Grundkörper (2) sowohl zumindest einen axialen Abschnitt (27, 29) des ersten Grundkörperteils (10) als auch jeweils zumindest einen axialen Abschnitt (34p) des zweiten Grundkörperteils (11) und des dritten Grundkörperteils (12) umschließt.

5. Sicherheitskanülenanordnung (1) nach einem der Ansprüche 1 bis 4, **dadurch gekennzeichnet, dass** das erste Grundkörperteil (10) und das zweite Grundkörperteil (11) starr miteinander verbunden und einstückig ausgeformt sind.

6. Sicherheitskanülenanordnung (1) nach einem der Ansprüche 1 bis 4, **dadurch gekennzeichnet, dass** das erste Grundkörperteil (10) und das zweite Grundkörperteil (11) über ein Filmscharnier (13) miteinander verbunden sind, wobei eine Scharnierachse des Filmscharniers (13) senkrecht zu und in einem Abstand zu der Längsachse (7) des Gleitkörpers (15) ausgerichtet ist.

7. Sicherheitskanülenanordnung (1) nach einem der Ansprüche 1 bis 6, **dadurch gekennzeichnet, dass** das dritte Grundkörperteil (12) und das zweite Grundkörperteil (11) mittels eines Filmscharniers miteinander verbunden sind, dessen Scharnierachse parallel oder senkrecht zu der Längsachse (7) des Gleitkörpers (15) ausgerichtet ist.

8. Sicherheitskanülenanordnung (1) nach einem der Ansprüche 1 bis 6, **dadurch gekennzeichnet, dass** das dritte Grundkörperteil (12) und das erste Grundkörperteil (10) mittels eines Filmscharniers (14) miteinander verbunden sind, dessen Scharnierachse senkrecht zu und in einem Abstand zu der Längsachse (7) des Gleitkörpers (15) ausgerichtet ist.

9. Sicherheitskanülenanordnung (1) nach den Ansprüchen 6 und 8, **dadurch gekennzeichnet, dass** die beiden Filmschaniere (13, 14), in axiale Richtung betrachtet, denselben Abstand von der Spitze (18) der Kanüle (17) aufweisen.

10. Sicherheitskanülenanordnung (1) nach Anspruch 9, **dadurch gekennzeichnet, dass** die beiden Filmscharniere (13, 14) jeweils denselben Abstand zu der Längsachse (7) des Gleitkörpers (15) aufweisen.

11. Sicherheitskanülenanordnung (1) nach einem der Ansprüche 1 bis 10, **dadurch gekennzeichnet, dass** das erste Grundkörperteil (10)
- in einem distalen Abschnitt (26) rotationssymmetrisch und/oder
- in einem proximalen Abschnitt (27) quaderförmig ausgebildet ist, wobei vorzugsweise an gegenüber liegenden proximalen Kanten des quaderförmigen proximalen Abschnitts (27) jeweils ein Filmscharnier (13, 14) angeordnet ist.

12. Sicherheitskanülenanordnung (1) nach Anspruch 11, **dadurch gekennzeichnet, dass** der rotationssymmetrische Abschnitt (26) des ersten Grundkörperteils (10) einen zylindrischen Spitzenabschnitt (28) und einen ebenfalls vorzugsweise zylindrischen Übergangsabschnitt (29) zur Aufnahme des in der Gebrauchsstellung komprimierten Stellelements (21) besitzt, wobei vorzugsweise auf den Spitzenabschnitt (28) ein abziehbarer rohrförmiger Kanülenschutz (4) aufgeschoben ist.

13. Sicherheitskanülenanordnung (1) nach einem der Ansprüche 1 bis 12, **dadurch gekennzeichnet, dass** das zweite Grundkörperteil (10) und das dritte Grundkörperteil (11) in einem zusammengefügten Zustand gemeinsam
- einen sich in proximale Richtung an die Filmscharniere (13, 14) anschließenden Griffbereich (9) ausbilden, der vorzugsweise auf seinem Außenmantel mit Rippen (6, 8) oder einer Vielzahl von Erhebungen zur Erhöhung der Griffigkeit ausgestattet ist und/oder
- einen sich in proximale Richtung an den Griffbereich (9) anschließenden Auslösebereich (36) ausbilden, der einen Auslösemechanismus (37) aufweist und/oder
- einen an einem proximalen Ende des Grundkörpers (2) angeordneten Verschlussbereich (38) ausbilden, in dem das zweite Grundkörperteil (11) und das dritte Grundkörperteil (12) mittels Schnapphaken (39) und/oder Rastnasen formschlüssig miteinander verbunden sind.

14. Sicherheitskanülenanordnung (1) nach einem der Ansprüche 1 bis 13, **dadurch gekennzeichnet, dass** das erste Grundkörperteil (10) ein distales Ende des Grundkörpers (2) bildet.

## Claims

1. A safety cannula assembly (1) comprising
a) a cannula (17) for puncturing human or animal tissue, wherein the cannula has, in a distal end section, a tip (18),
b) a sliding body (15) which, in a distal end section (49), is provided with the cannula (17) and, in a proximal end section (46), with a flexible tube (5), wherein a flow connection extending through the sliding body (15) exists between the tip (18) of the cannula (17) and a proximal end of the tube (5),
c) a base body (2), in which the sliding body (15) is displaceable from a use position, in which the tip (18) of the cannula (17) is located outside the base body (2), into a safety position, in which the tip (18) of the cannula (17) is located inside the base body (2), wherein the base body (2) is composed of at least three base body parts (10, 11, 12), of which
- a first base body part (10) is designed in the form of a sleeve and surrounds a distal end section (49) of the sliding body (15) in its use position,
- a second base body part (11) adjoins the first base body part (10) in the proximal direction and is connected thereto, wherein the second base body part (11) is formed in a U-shaped, L-shaped, C-shaped or cover-shaped manner in cross-section and
- a third base body part (12) and the second base body part (11) can be assembled to form a hollow body open at both opposite end faces, wherein the third base body part (12) is formed in a cover-shaped, C-shaped, L-shaped or U-shaped manner and is connected to the second base body part (11) or to the first base body part (10) via a film hinge (13, 14),
d) an actuating element (21) arranged between the base body (2) and the sliding body (15), by means of which the sliding body (15) can be displaced from the use position into the safety position, and
e) a release mechanism (37) arranged on the base body (2), by means of which the displacement of the sliding body (15) from the use position into the safety position can be triggered, wherein the release mechanism (37) has at least one release member (67a, 67b) which can be acted upon by a person using the safety cannula assembly (1) with a radially directed pressing force or with a torque and which is provided with a locking element (61a, 61b) which, as a result of the pressing force or torque being applied, is displaceable from a locking position, in which it engages with the sliding body (15) and locks it in the use position, into a release position, in which it is disengaged from the sliding body (15) in such a manner that the sliding body moves into the safety position,
**characterized in that** the actuating element (21) is supported with its distal end against a support surface (21s) of the first base body part (10).

2. The safety cannula assembly (1) according to claim 1, **characterized in that** the base body (2) is provided on each of two opposite sides with a grip wing (31).

3. The safety cannula assembly (1) according to claim 1 or 2, **characterized in that** the actuating element (21), with its distal end, is supported against a step formed in an interior space of the first base body part (10) or against an end wall forming a distal end of the first base body part (10).

4. The safety cannula assembly (1) according to any one of claims 1 to 3, **characterized in that** the grip wings (31) are connected to a sleeve-shaped connecting part (30) which is pushed onto the base body (2), wherein preferably an overlap region (34) between the connecting part (30) and the base body (2) encloses both at least one axial section (27, 29) of the first base body part (10) and in each case at least one axial section (34p) of the second base body part (11) and of the third base body part (12).

5. The safety cannula assembly (1) according to any one of claims 1 to 4, **characterized in that** the first base body part (10) and the second base body part (11) are rigidly connected to one another and integrally formed.

6. The safety cannula assembly (1) according to any one of claims 1 to 4, **characterized in that** the first base body part (10) and the second base body part (11) are connected to one another via a film hinge (13), wherein a hinge axis of the film hinge (13) is oriented perpendicularly to, and spaced apart from, the longitudinal axis (7) of the sliding body (15).

7. The safety cannula assembly (1) according to any one of claims 1 to 6, **characterized in that** the third base body part (12) and the second base body part (11) are connected to one another by means of a film hinge, the hinge axis of which is oriented parallel to or perpendicular to the longitudinal axis (7) of the sliding body (15).

8. The safety cannula assembly (1) according to any one of claims 1 to 6, **characterized in that** the third base body part (12) and the first base body part (10) are connected to one another by means of a film hinge (14), the hinge axis of which is oriented perpendicularly to, and spaced apart from, the longitudinal axis (7) of the sliding body (15).

9. The safety cannula assembly (1) according to claims 6 and 8, **characterized in that** the two film hinges (13, 14), viewed in the axial direction, are at the same distance from the tip (18) of the cannula (17).

10. The safety cannula assembly (1) according to claim 9, **characterized in that** the two film hinges (13, 14) are each at the same distance from the longitudinal axis (7) of the sliding body (15).

11. The safety cannula assembly (1) according to any one of claims 1 to 10, **characterized in that** the first base body part (10)
- is rotationally symmetrical in a distal section (26), and/or
- is cuboid-shaped in a proximal section (27), wherein preferably a film hinge (13, 14) is arranged in each case on opposite proximal edges of the cuboid-shaped proximal section (27).

12. The safety cannula assembly (1) according to claim 11, **characterized in that** the rotationally symmetrical section (26) of the first base body part (10) has a cylindrical tip section (28) and a preferably likewise cylindrical transition section (29) for receiving the actuating element (21) compressed in the use position, wherein preferably a removable tubular cannula protector (4) is pushed onto the tip section (28).

13. The safety cannula assembly (1) according to any one of claims 1 to 12, **characterized in that** the second base body part (11) and the third base body part (12), in an assembled state, together form
- a grip region (9) adjoining the film hinges (13, 14) in the proximal direction, which is preferably provided on its outer surface with ribs (6, 8) or with a plurality of projections for increasing grip, and/or
- a release region (36) adjoining the grip region (9) in the proximal direction, which comprises a release mechanism (37), and/or
- a closure region (38) arranged at a proximal end of the base body (2), in which the second base body part (11) and the third base body part (12) are connected to one another by means of snap hooks (39) and/or latching projections in a form-fitting manner.

14. The safety cannula assembly (1) according to any one of claims 1 to 13, **characterized in that** the first base body part (10) forms a distal end of the base body (2).

## Revendications

1. Système de canule de sécurité (1) comprenant
a) une canule (17) pour la ponction de tissu humain ou animal, sachant que la canule comporte une pointe (18) dans une section finale distale,
b) un corps coulissant (15) qui est doté dans une section finale distale (49) de la canule (17) et dans une section finale proximale (46) d'un tuyau flexible (5), sachant qu'il existe une liaison d'écoulement s'étendant à travers le corps coulissant (15) entre la pointe (18) de la canule (17) et une extrémité proximale du tuyau (5),
c) un corps de base (2), dans lequel le corps coulissant (15) peut être déplacé d'une position d'utilisation, dans laquelle se trouve la pointe (18) de la canule (17) en dehors du corps de base (2), à une position de sécurisation, dans laquelle se trouve la pointe (18) de la canule (17) à l'intérieur du corps de base (2), sachant que le corps de base (2) est composé d'au moins trois parties de corps de base (10, 11, 12), dont
- une première partie de corps de base (10) est constituée en forme de manchon et entoure une section finale distale (49) du corps coulissant (15) dans la position d'utilisation de celle-ci,
- une deuxième partie de corps de base (11) se raccorde en direction proximale à la première partie de corps de base (10) et est reliée à celle-ci, sachant que la deuxième partie de corps de base (11) est constituée en section en forme de U, en forme de L, en forme de C ou en forme de couvercle et
- une troisième partie de corps de base (12) et la deuxième partie de corps de base (11) peuvent être assemblées en un corps creux ouvert sur les côtés frontaux opposés, sachant que la troisième partie de corps de base (12) est constituée en forme de couvercle, en forme de C, en forme de L ou en forme de U et est reliée à la deuxième partie de corps de base (11) ou à la première partie de corps de base (10) par le biais d'une charnière à film (13, 14),
d) un élément de réglage (21) disposé entre le corps de base (2) et le corps coulissant (15) au moyen duquel le corps coulissant (15) peut être déplacé de la position d'utilisation à la position de sécurisation, et
e) un mécanisme de déclenchement (37) disposé sur le corps de base (2) au moyen duquel le déplacement du corps coulissant (15) peut être déclenché de la position d'utilisation à la position de sécurisation, sachant que le mécanisme de déclenchement (37) comporte au moins un organe de déclenchement (67a, 67b), qui peut être sollicité par une personne utilisant le système de canule de sécurité (1) avec une force de pression dirigée radialement ou avec un couple et qui est doté d'un élément de verrouillage (61a, 61b), qui peut être déplacé en raison d'une sollicitation avec la force de pression ou le couple d'une position de verrouillage, dans laquelle il est en prise avec le corps coulissant (15) et verrouille celui-ci dans la position d'utilisation, à une position de déclenchement, dans laquelle il est hors de prise avec le corps coulissant (15) de telle sorte que celui-ci se rend dans la position de sécurisation,
**caractérisé en ce que** l'élément de réglage (21) s'appuie avec son extrémité distale sur une surface d'appui (21s) de la première partie de corps de base (10).

2. Système de canule de sécurité (1) selon la revendication 1, **caractérisé en ce que** le corps de base (2) est doté respectivement sur deux côtés opposés d'une ailette de préhension (31).

3. Système de canule de sécurité (1) selon la revendication 1 ou 2, **caractérisé en ce que** l'élément de réglage (21) s'appuie avec son extrémité distale sur un gradin constitué dans un espace intérieur de la première partie de corps de base (10) ou une paroi frontale formant une terminaison distale de la première partie de corps de base (10).

4. Système de canule de sécurité (1) selon l'une quelconque des revendications 1 à 3, **caractérisé en ce que** les ailettes de préhension (31) sont raccordées à une pièce de liaison en forme de manchon (30), qui est glissée sur le corps de base (2), sachant de préférence qu'une zone de chevauchement (34) entre la pièce de liaison (30) et le corps de base (2) entoure aussi bien au moins une section axiale (27, 29) de la première partie de corps de base (10) que respectivement au moins une section axiale (34p) de la deuxième partie de corps de base (11) et de la troisième partie de corps de base (12).

5. Système de canule de sécurité (1) selon l'une quelconque des revendications 1 à 4, **caractérisé en ce que** la première partie de corps de base (10) et la deuxième partie de corps de base (11) sont fermement reliées entre elles et formées en une seule pièce.

6. Système de canule de sécurité (1) selon l'une quelconque des revendications 1 à 4, **caractérisé en ce que** la première partie de corps de base (10) et la deuxième partie de corps de base (11) sont reliées entre elles par le biais d'une charnière à film (13), sachant qu'un axe de charnière de la charnière à film (13) est orienté perpendiculairement à et à distance de l'axe longitudinal (7) du corps coulissant (15).

7. Système de canule de sécurité (1) selon l'une quelconque des revendications 1 à 6, **caractérisé en ce que** la troisième partie de corps de base (12) et la deuxième partie de corps de base (11) sont reliées entre elles au moyen d'une charnière à film, dont l'axe de charnière est orienté parallèlement ou perpendiculairement à l'axe longitudinal (7) du corps coulissant (15).

8. Système de canule de sécurité (1) selon l'une quelconque des revendications 1 à 6, **caractérisé en ce que** la troisième partie de corps de base (12) et la première partie de corps de base (10) sont reliées entre elles au moyen d'une charnière à film (14) dont l'axe de charnière est orienté perpendiculairement à et à une distance de l'axe longitudinal (7) du corps coulissant (15).

9. Système de canule de sécurité (1) selon les revendications 6 et 8, **caractérisé en ce que** les deux charnières à film (13, 14), vues dans la direction axiale, comportent la même distance par rapport à la pointe (18) de la canule (17).

10. Système de canule de sécurité (1) selon la revendication 9, **caractérisé en ce que** les deux charnières à film (13, 14) comportent respectivement la même distance par rapport à l'axe longitudinal (7) du corps coulissant (15).

11. Système de canule de sécurité (1) selon l'une quelconque des revendications 1 à 10, **caractérisé en ce que** la première partie de corps de base (10) est constituée
- symétrique en rotation dans une section distale (26), et/ou
- de forme parallélépipédique dans une section proximale (27), sachant de préférence qu'une charnière à film (13, 14) est respectivement disposée sur les bords opposés de la section proximale parallélépipédique (27).

12. Système de canule de sécurité (1) selon la revendication 11, **caractérisé en ce que** la section symétrique en rotation (26) de la première partie de corps de base (10) possède une section de pointe (28) cylindrique et également de préférence une section de transfert (29) cylindrique pour recevoir l'élément de réglage (21) comprimé à l'état d'utilisation, sachant de préférence qu'une protection de canule (4) tubulaire enlevable est glissée sur la section de pointe (28).

13. Système de canule de sécurité (1) selon l'une quelconque des revendications 1 à 12, **caractérisé en ce que** la deuxième partie de corps de base (11) et la troisième partie de corps de base (12) constituent en commun dans un état assemblé
- une zone de préhension (9) se raccordant en direction proximale aux charnières à film (13, 14), qui est dotée de préférence sur son enveloppe extérieure de cannelures (6, 8) ou d'une pluralité de bossages pour augmenter l'adhérence et/ou
- constituent une zone de libération (36) se raccordant en direction proximale à la zone de préhension (9), qui comporte un mécanisme de libération (37), et/ou
- constituent une zone de fermeture (38) disposée sur une extrémité proximale du corps de base (2) dans laquelle la deuxième partie de corps de base (11) et la troisième partie de corps de base (12) sont reliées entre elles par conformité de forme au moyen de crochets d'encliquetage (39) et/ou de becs d'encliquetage.

14. Système de canule de sécurité (1) selon l'une quelconque des revendications 1 à 13, **caractérisé en ce que** la première partie de corps de base (10) forme une extrémité distale du corps de base (2).
